# FASCICULE DE BREVET EUROPEEN

(11) **EP 3 615 500 B1**
(45) Date de publication et mention de la délivrance du brevet: **24.03.2021**
(21) Numéro de dépôt: 18725274.7
(22) Date de dépôt: 20.04.2018
(51) Int. Cl.: C07C 17/20, C07C 17/25, C07C 17/383, C07C 17/38, C07C 17/386, C07C 21/18

(54) **PROCEDE DE PRODUCTION ET DE PURIFICATION DU 2,3,3,3-TETRAFLUOROPROPENE**
VERFAHREN ZUR HERSTELLUNG UND REINIGUNG VON 2,3,3,3-TETRAFLUORPROPEN
METHOD FOR THE PRODUCTION AND PURIFICATION OF 2,3,3,3-TETRAFLUOROPROPENE

(30) Priorité: 28.04.2017 FR 1753744
(43) Date de publication de la demande: 04.03.2020
(73) Titulaire: Arkema France, 92700 Colombes (FR)
(72) Inventeur: DEUR-BERT, Dominique, 69390 Charly (FR); PIGAMO, Anne, 69340 Francheville (FR); WENDLINGER, Laurent, 69510 Soucieu en Jarrest (FR)
(74) Mandataire: Arkema Patent
(86) Numéro de dépôt international: PCT/FR2018/050995
(87) Numéro de publication internationale: WO 2018/197788

(56) Documents cités:
- WO-A1-2013/088195
- US-A1- 2011 160 499

## Description

### Domaine technique de l'invention

L'invention se rapporte à un procédé de purification du 2,3,3,3-tetrafluoro-1-propène. En outre, l'invention se rapporte également à un procédé de production et de purification du 2,3,3,3-tetrafluoro-1-propène.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et/ou de dehydrohalogénation. Ce type de réaction est effectué en phase gazeuse et génère des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propène (HCFO-1233zd), 1,3,3,3-tetrafluoro-1-propène (HFO-1234ze) et le 1,1,1,3,3-pentafluoropropane (HFC-245fa) sont observées. Ces impuretés sont des isomères des composés principaux visant à être obtenues par le procédé de production du 2,3,3,3-tétrafluoro-1-propène outre ce dernier, i.e. 2-chloro-3,3,3-trifluoro-1-propène (HCFO-1233xf) et 1,1,1,2,2-pentafluoropropane (HFC-245cb). Compte tenu des points d'ébullition respectifs du 1-chloro-3,3,3-trifluoro-1-propène (HCFO-1233zd), 1,3,3,3-tetrafluoro-1-propène (HFO-1234ze) et le 1,1,1,3,3-pentafluoropropane (HFC-245fa), ceux-ci peuvent s'accumuler dans la boucle réactionnelle et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proches ou lorsque ceux-ci forment des compositions azéotropes ou quasi-azéotropes, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (HFC-245fa) et 1-chloro-3,3,3-trifluoro-trans-1-propène (HCFO-1233zd) par distillation en présence d'un solvant ayant un point d'ébullition supérieur à celui de 1-chloro-3,3,3-trifluoro-trans-1-propène.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C. Comme mentionné par WO 98/19982, la sélection de l'agent d'extraction peut s'avérer complexe en fonction des produits à séparer. On connaît par WO 2013/088195 un procédé de préparation de 2,3,3,3-tetrafluoropropène à partir de 1,1,1,2,3-pentachloropropane et/ou 1,1,2,2,3-pentachloropropane. On connait également par US 2011/0160499 un procédé de production du 2,3,3,3-tétrafluoropropène à partir du 1,1,1,2,3-pentachloropropane. Il existe donc toujours un besoin pour la mise en œuvre d'un procédé particulier de purification du 2,3,3,3-tetrafluoro-1-propène.

### Résumé de l'invention

Dans un procédé de production du 2,3,3,3-tetrafluoro-1-propène, le choix de conditions opérationnelles particulières peut favoriser la présence de certaines impuretés ou d'isomères de celles-ci. La présence d'impuretés telles que 1,3,3,3-tetrafluoro-1-propène (HFO-1234ze) peut être observée tout comme celle de 1-chloro-3,3,3-trifluoro-1-propène (HCFO-1233zd), et 1,1,1,3,3-pentafluoropropane (HFC-245fa). Des composés générant des impuretés de type propyne halogénées comme la trifluoropropyne, chlorotrifluoropropyne ou la tetrafluoropropyne ont également été identifiés. Les impuretés peuvent provenir de réactions secondaires induites par des composés produits intermédiairement pendant la production du 2,3,3,3-tetrafluoropropène, et peuvent présenter des propriétés physiques telles que leur élimination peut s'avérer complexe. Certaines impuretés, telles que les propynes halogénées peuvent favoriser la formation de coke et aboutir à une désactivation du catalyseur. Il y a donc un besoin pour fournir un procédé limitant la présence des impuretés telles que des propynes halogénées et des composés dont elles sont issues. La présente invention permet notamment la production de 2,3,3,3-tetrafluoro-1-propène avec une pureté améliorée.

Selon un premier aspect, l'invention fournit un procédé de production et de purification du 2,3,3,3-tétrafluoropropène (HFO-1234yf) mis en œuvre à partir d'une composition de départ comprenant au moins un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl; ledit procédé comprenant les étapes de :
a) mise en contact, en présence d'un catalyseur, de la composition de départ avec HF dans des conditions efficaces pour produire une composition **A** comprenant HCl, une partie du HF n'ayant pas réagi, 2,3,3,3-tétrafluoropropène (HFO-1234yf), des produits intermédiaires **B** consistant en 2-chloro-3,3,3-trifluoropropène (HCFO-1233xf), 1,1,1,2,2-pentafluoropropane (HFC-245cb), trans-1,3,3,3-tetrafluoro-1-propène (HFO-1234zeE) et des produits secondaires **C** consistant en trans-1-chloro-3,3,3-trifluoro-1-propène (HCFO-1233zdE), cis-1-chloro-3,3,3-trifluoro-1-propène (HCFO-1233zdZ), cis-1,3,3,3-tetrafluoro-1-propène (HFO-1234zeZ), 1,1,1,3,3-pentafluoropropane (HFC-245fa), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), 1,1,1,3,3,3-hexafluoropropane (HFC-236fa) et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w = 0, w étant le nombre d'insaturations dans le composé de formule (II) considéré ;
b) récupération de ladite composition **A** et purification, de préférence distillation, de celle-ci pour former et récupérer un courant gazeux **G1** comprenant HCl, 2,3,3,3-tétrafluoropropène (HFO-1234yf), une partie du HF n'ayant pas réagi, une partie des produits intermédiaires **B** et une partie des produits secondaires **C** ; et un courant, de préférence liquide, **L1** comprenant une partie du HF n'ayant pas réagi, une partie des produits intermédiaires **B** et une partie des produits secondaires **C** ;
caractérisé en ce que ledit courant gazeux **G1** purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (HFO-1234yf), une partie du HF n'ayant pas réagi, ladite une partie des produits intermédiaires **B** et ladite une partie des produits secondaires **C,** avantageusement en bas de colonne de distillation ;
b2-1) distillation dudit courant **G1b** obtenu à l'étape b1) dans des conditions efficaces pour former un courant **G1c** gazeux comprenant 2,3,3,3-tétrafluoropropène (HFO-1234yf), une portion de ladite partie dudit HF n'ayant pas réagi, une portion de ladite partie des produits intermédiaires **B**, avantageusement en tête de colonne de distillation, et un courant **G1d** liquide comprenant une portion de ladite partie des produits intermédiaires **B** et ladite partie des produits secondaires **C** et une portion de ladite partie dudit HF n'ayant pas réagi, avantageusement en bas de colonne de distillation ;
b2-2) distillation dudit courant **G1d** obtenu à l'étape b2-1) dans des conditions efficaces pour former un courant **G1d'** comprenant ladite portion de ladite partie des produits intermédiaires **B**, ladite portion de ladite partie dudit HF n'ayant pas réagi et une portion de ladite partie des produits secondaires **C** comprenant cis-1,3,3,3-tetrafluoro-1-propène (HFO-1234zeZ), 1,1,1,3,3,3-hexafluoropropane (HFC-236fa) et une partie de trans-1-chloro-3,3,3-trifluoro-1-propène (HCFO-1233zdE) et 1,1,1,3,3-pentafluoropropane (HFC-245fa); et un courant **G1d"** comprenant une portion de ladite partie des produits secondaires **C** comprenant cis-1-chloro-3,3,3-trifluoro-1-propène (HCFO-1233zdZ), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w est 0, w étant le nombre d'insaturations dans le composé de formule (II) considéré.

Le nombre d'insaturations correspond au nombre de double liaison carbone-carbone dans le composé de formule (II).

Selon un mode de réalisation préféré, au moins 90% du 1,1,1,2,2-pentafluoropropane et du trans-1,3,3,3-tetrafluoro-1-propène contenus dans le courant **G1d** sont récupérés dans le courant **G1d',** avantageusement au moins 91%, de préférence au moins 92%, plus préférentiellement au moins 93%, en particulier au moins 94%, plus particulièrement au moins 95%, de manière privilégiée au moins 96%, de manière préférentiellement privilégiée au moins 97%, de manière particulièrement privilégiée au moins 98% du 1,1,1,2,2-pentafluoropropane et du trans-1,3,3,3-tetrafluoropropène contenus dans le courant **G1d** sont récupérés dans le courant **G1d'**.

Selon un mode de réalisation préféré, le courant **G1d'** comprend de 60 à 70% en poids de 1,1,1,2,2-pentafluoropropane, de 1% à 5% en poids de trans-1,3,3,3-tetrafluoropropène et 10 à 15% en poids de 2-chloro-3,3,3-trifluoropropène sur base du poids total du courant **G1d'**.

Selon un mode de réalisation préféré, au moins 85% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d'**, avantageusement au moins 90%, de préférence au moins 92%, plus préférentiellement au moins 93%, en particulier au moins 95%, plus particulièrement au moins 97% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d'**.

Selon un mode de réalisation préféré, le courant **G1d'** comprend de 0,1 à 0,5% en poids de cis-1,3,3,3-tetrafluoro-1-propène (HFO-1234zeZ), de 1,0% à 5,0% en poids de 1,1,1,3,3,3-hexafluoropropane (HFC-236fa), 0,5 à 2,0% en poids de trans-1-chloro-3,3,3-trifluoro-1-propène (HCFO-1233zdE) et de 0,1 à 1,0% en poids de 1,1,1,3,3-pentafluoropropane (HFC-245fa) sur base du poids total du courant **G1d'**.

Selon un mode de réalisation préféré, le courant **G1d'** comprend également 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe) en une teneur totale inférieure à 5% en poids sur base du poids total de **G1d',** avantageusement inférieure à 4% en poids, de préférence inférieure à 3% en poids, plus préférentiellement inférieure à 2% en poids, en particulier inférieure à 1% en poids, plus particulièrement inférieure à 0,5% en poids sur base du poids total de **G1d'**.

Selon un mode de réalisation préféré, le courant **G1d'** est recyclé à l'étape a).

Selon un mode de réalisation préféré, le procédé comprend également les étapes :
b3) mise en contact du courant **G1c** avec une solution aqueuse d'acide fluorhydrique de concentration supérieure à 40% pour former un courant **G1c'** diphasique comprenant 2,3,3,3-tétrafluoropropène (HFO-1234yf), de l'acide fluorhydrique, une portion de ladite partie des produits intermédiaires **B** et une portion de ladite partie des produits secondaires **C**,
b4) stockage dudit courant **G1c'** diphasique dans un réservoir tampon, ledit second courant diphasique étant constitué d'une phase liquide et d'une phase gazeuse,
b5) passage de ladite phase gazeuse dudit courant **G1c'** dans une colonne d'absorption alimenté à contre-courant par un flux aqueux pour former un courant **G1c"** comprenant 2,3,3,3-tétrafluoropropène (HFO-1234yf), une portion de ladite partie des produits intermédiaires **B** et une portion de ladite partie des produits secondaires C et un courant **G1c'''** comprenant HF ;
et optionnellement les étapes :
b6) neutralisation dudit courant **G1c"** obtenu à l'étape b5) par une solution aqueuse alcaline pour former un courant neutralisé, et
b7) séchage dudit courant neutralisé obtenu à l'étape b6), de préférence sur tamis moléculaire, pour former un courant neutralisé et séché **G1c""**.

Selon un mode de réalisation préféré, la solution aqueuse d'acide fluorhydrique utilisée à l'étape b3) est à une température comprise entre 0 à 30°C avant sa mise en contact avec le courant **G1c.**

Selon un mode de réalisation préféré, le procédé comprend une étape c), subséquente à l'étape b5) ou à l'étape b7), dans laquelle le courant **G1c"** obtenu à l'étape b5) ou le courant **G1c""** obtenu à l'étape b7) comprend 2,3,3,3-tétrafluoropropène (HFO-1234yf), 1,1,1,2,2-pentafluoropropane (HFC-245cb) et trans-1,3,3,3-tetrafluoro-1-propène (HFO-1234zeE) ; et ledit courant **G1c" ou G1c""** est distillé pour former un courant **G1e'** comprenant 2,3,3,3-tétrafluoropropène (HFO-1234yf) et 1,1,1,2,2-pentafluoropropane (HFC-245cb) et un courant **G1h'** comprenant trans-1,3,3,3-tetrafluoro-1-propène (HFO-1234zeE) ;
avantageusement le courant **G1c"** ou **G1c""** est distillé par distillation extractive ;
de préférence le courant **G1c"** ou **G1c""** est distillé par distillation extractive par les étapes :
c1') mise en contact dudit courant **G1c" ou G1c""** avec un agent d'extraction organique pour former un courant **G1g',** et
c2') distillation extractive du courant **G1g'** pour former le flux **G1e'** comprenant 2,3,3,3-tetrafluoropropène (HFO-1234yf) et 1,1,1,2,2-pentafluoropropane (HFC-245cb), avantageusement en tête de colonne de distillation, et le courant **G1h'** comprenant trans-1,3,3,3-tetrafluoro-1-propène (HFO-1234zeE) et ledit agent d'extraction organique, avantageusement en bas de colonne de distillation.

Selon un mode de réalisation préféré, ledit courant liquide **L1** comprend une partie des produits intermédiaires **B** et tout ou partie des produits secondaires C, et une partie du courant liquide **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C** ; optionnellement ou non, ledit courant **G1d** formé à l'étape b2) est mélangé au courant liquide **L1** avant que ce dernier soit porté à basse température, avantageusement ladite première phase **L1a** est recyclée à l'étape a) ;
avantageusement ladite seconde phase **L1b** est distillée pour récupérer un courant **L1c** comprenant 1,1,1,2,2-pentafluoropropane (HFC-245cb) et trans-1,3,3,3-tetrafluoro-1-propène (HFO-1234zeE), avantageusement en tête de colonne de distillation, et un courant **L1d** comprenant 2-chloro-3,3,3-trifluoro-1-propène (HCFO-1233xf), E-1-chloro-3,3,3-trifluoro-1-propène (HCFO-1233zdE) et 1,1,1,3,3-pentafluoropropane (HFC-245fa) ; avantageusement en bas de colonne de distillation, avantageusement ledit courant **L1c** est recyclé à l'étape a) ;
de préférence ledit courant **L1d** est séparé par distillation extractive pour former un flux comprenant 2-chloro-3,3,3-trifluoro-1-propène (HCFO-1233xf) et un courant comprenant E-1-chloro-3,3,3-trifluoro-1-propène (HCFO-1233zdE) et 1,1,1,3,3-pentafluoropropane (HFC-245fa).

### Brève description des dessins

La figure 1 représente schématiquement un dispositif mettant en œuvre un procédé de production du 2,3,3,3-tetrafluoro-1-propène selon un mode de réalisation particulier de la présente invention.
Les figures 2, 3a, 3b et 3c représentent schématiquement une partie du dispositif mettant en œuvre la purification du 2,3,3,3-tetrafluoro-1-propène selon un autre mode de réalisation particulier de la présente invention.

### Description détaillée de l'invention

La présente invention permet la production et la purification du 2,3,3,3-tétrafluoropropène (HFO-1234yf). Selon un premier aspect de la présente invention, un procédé de production et de purification du 2,3,3,3-tétrafluoropropène (HFO-1234yf) est fourni. Ledit procédé est mis en œuvre à partir d'une composition de départ comprenant au moins un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl.

De préférence, ledit procédé comprend l'étape a) de mise en contact, en présence d'un catalyseur, de la composition de départ avec HF dans des conditions efficaces pour produire une composition **A** comprenant HCl, une partie du HF n'ayant pas réagi, 2,3,3,3-tétrafluoropropène (HFO-1234yf), des produits intermédiaires **B** consistant en 2-chloro-3,3,3-trifluoropropène (HCFO-1233xf), 1,1,1,2,2-pentafluoropropane (HFC-245cb), trans-1,3,3,3-tetrafluoro-1-propène (HFO-1234zeE) et des produits secondaires **C** consistant en trans-1-chloro-3,3,3-trifluoro-1-propène (HCFO-1233zdE), cis-1-chloro-3,3,3-trifluoro-1-propène (HCFO-1233zdZ), cis-1,3,3,3-tetrafluoro-1-propène (HFO-1234zeZ), 1,1,1,3,3-pentafluoropropane (HFC-245fa), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), 1,1,1,3,3,3-hexafluoropropane (HFC-236fa) et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w = 0, w étant le nombre d'insaturations dans le composé de formule (II) considéré.

Lesdits produits secondaires **C** peuvent également comprendre un ou plus isomères du chloropentafluoropropane (HCFC-235) autre que le 2-chloro-1,1,1,3,3-pentafluoropropane (HCFC-235da). Lesdits produits secondaires **C** peuvent également comprendre un ou plus isomères du dichlorotrifluoropropène (HCFO-1223) autre que le 1,2-dichloro-3,3,3-trifluoropropène (HCFO-1223xd). Lesdits produits secondaires **C** peuvent également comprendre un ou plus isomères du chlorotetrafluoropropène (HCFO-1224) autre que le 2-chloro-1,3,3,3-tetrafluoropropène (HCFO-1224xe).

De préférence, lesdits produits secondaires **C** peuvent également comprendre un ou plusieurs composés de formule (II) CₙHₓF_{y}Cl_{z} dans laquelle n = 4, 5 ou 6, x est un nombre entier de 0 à 4, y est un nombre entier de 6 à 12, z est un nombre entier de 0 à 2, avec 2n = x+y+z si w est 1 ou 2n-2 = x+y+z si w est 2 ou 2n+2 = x+y+z si w = 0, w étant le nombre d'insaturations dans le composé de formule (II) considéré.

En particulier, les composés de formule (II) sont sélectionnés parmi le groupe consistant en hexafluorobutene (HFO-1336), chlorohexafluorobutene (HCFO-1326), heptafluorobutene (HFO-1327), octafluorobutane (HFC-338), dichlorooctafluoropentene (HCFO-1418), chlorononafluoropentene (HCFO-1419), chlorooctafluoropentene (HCFO-1428), nonafluoropentene (HFO-1429), octafluoropentene (HFO-1438), heptafluoropentene (HFO-1447), chlorononafluorohexene (HCFO-1539), chloroheptafluorohexene (HCFO-1557), decafluorohexene, undecafluorohexene, chlorooctafluorohexadiene (HCFO-2528). Les composés de formule (II) peuvent exister sous différentes formes isomériques qui sont englobés dans la présente demande.

Ladite composition produite à l'étape a) est récupérée et purifiée. De préférence, la purification est effectuée par distillation.

De préférence, la purification de ladite composition **A** effectuée à l'étape b) comprend la distillation de ladite composition **A** pour récupérer en tête de colonne de distillation un courant gazeux **G1** comprenant HCl, une partie du HF n'ayant pas réagi et 2,3,3,3-tétrafluoropropène (HFO-1234yf); et en bas de colonne de distillation un courant liquide **L1** comprenant une partie du HF n'ayant pas réagi. Tout ou partie des produits intermédiaires **B** et tout ou partie des produits secondaires **C** peuvent être contenus dans ledit courant gazeux **G1** et/ou dans ledit courant liquide **L1**.

De préférence, tout ou partie du 1,1,1,2,2-pentafluoropropane (HFC-245cb) peut être contenu dans ledit courant gazeux **G1**. Tout ou partie du 1,1,1,2,2-pentafluoropropane (HFC-245cb) peut aussi être contenu dans ledit courant liquide **L1**.

De préférence, tout ou partie du trans-1,3,3,3-tetrafluoro-1-propène (HFO-1234zeE) peut être contenu dans ledit courant gazeux **G1**. Tout ou partie du trans-1,3,3,3-tetrafluoro-1-propène (HFO-1234zeE) peut aussi être contenu dans ledit courant liquide **L1**.

De préférence, tout ou partie du 2-chloro-3,3,3-trifluoropropène (HCFO-1233xf) peut être contenu dans ledit courant gazeux **G1**. Tout ou partie du 2-chloro-3,3,3-trifluoropropène (HCFO-1233xf) peut aussi être contenu dans ledit courant liquide **L1**. De manière privilégiée, une partie du 2-chloro-3,3,3-trifluoropropène (HCFO-1233xf) est contenu dans ledit courant liquide **L1,** avantageusement au moins 70%, au moins 75%, au moins 80%, au moins 85% ou au moins 90% du 2-chloro-3,3,3-trifluoropropène (HCFO-1233xf) présent initialement dans la composition **A** est contenu dans ledit courant liquide **L1**; le complément se trouvant dans ledit courant gazeux **G1**.

De préférence, tout ou partie du E-1-chloro-3,3,3-trifluoro-1-propène (HCFO-1233zdE) peut être contenu dans ledit courant gazeux **G1**. Tout ou partie du E-1-chloro-3,3,3-trifluoro-1-propène (HCFO-1233zdE) peut aussi être contenu dans ledit courant liquide **L1**. De manière privilégiée, une partie du E-1-chloro-3,3,3-trifluoro-1-propène (HCFO-1233zdE) est contenu dans ledit courant liquide **L1,** avantageusement au moins 70%, au moins 75%, au moins 80%, au moins 85% ou au moins 90% du 1-chloro-3,3,3-trifluoro-1-propène (HCFO-1233zd) présent initialement dans la composition A est contenu dans ledit courant liquide **L1**; le complément se trouvant dans ledit courant gazeux **G1**.

De préférence, tout ou partie du 1,1,1,3,3-pentafluoropropane (HFC-245fa) peut être contenu dans ledit courant gazeux **G1**. Tout ou partie du 1,1,1,3,3-pentafluoropropane (HFC-245fa) peut aussi être contenu dans ledit courant liquide **L1**. De manière privilégiée, une partie du 1,1,1,3,3-pentafluoropropane (HFC-245fa) est contenu dans ledit courant liquide **L1**, avantageusement au moins 70%, au moins 75%, au moins 80%, au moins 85% ou au moins 90% du 1,1,1,3,3-pentafluoropropane (HFC-245fa) présent initialement dans la composition **A** est contenu dans ledit courant liquide **L1**; le complément se trouvant dans ledit courant gazeux **G1**.

De préférence, ledit courant liquide **L1** comprend au moins 70% en mole d'acide fluorhydrique, avantageusement au moins 75% en mole d'acide fluorhydrique, de préférence au moins 80% en mole d'acide fluorhydrique, plus préférentiellement au moins 85% en mole d'acide fluorhydrique, en particulier au moins 90% en mole d'acide fluorhydrique.

En particulier, la récupération de ladite composition **A** et sa purification, de préférence par distillation, permet de former et de récupérer un courant gazeux **G1** comprenant HCl, 2,3,3,3-tétrafluoropropène (HFO-1234yf), une partie du HF n'ayant pas réagi, une partie des produits intermédiaires **B** et une partie des produits secondaires **C**, de préférence en tête de colonne de distillation ; et un courant liquide **L1** comprenant une partie du HF n'ayant pas réagi, une partie des produits intermédiaires **B** et une partie des produits secondaires **C**, de préférence en pied de colonne de distillation.

L'étape b) du présent procédé peut être effectuée par distillation à une pression de 2 à 8 bara, avantageusement de 3 à 6 bara, de préférence de 3,5 à 5,5 bara, en particulier à une pression de 4 bara. L'étape b) du présent procédé peut être effectuée par distillation de sorte à obtenir une température en tête de colonne de distillation de -30°C à 20°C, avantageusement de -20°C à 10°C, de préférence de -15°C à 0°C. L'étape b) du présent procédé peut être effectuée par distillation de sorte à obtenir une température en pied de colonne de distillation de 10°C à 100°C, avantageusement de 20°C à 90°C, de préférence de 30°C à 80°C, en particulier de 40 à 70°C.

Selon un mode de réalisation préféré, ledit premier courant est un courant gazeux **G1** purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (HFO-1234yf), une partie du HF n'ayant pas réagi, ladite une partie des produits intermédiaires **B** et ladite une partie des produits secondaires C, avantageusement en pied de colonne de distillation ;
b2-1) distillation dudit courant **G1b** obtenu à l'étape b1) dans des conditions efficaces pour former un courant **G1c** gazeux comprenant 2,3,3,3-tétrafluoropropène (HFO-1234yf), une portion de ladite partie dudit HF n'ayant pas réagi, une portion de ladite partie des produits intermédiaires **B**, avantageusement en tête de colonne de distillation, et un courant **G1d** liquide comprenant une portion de ladite partie des produits intermédiaires **B** et ladite partie des produits secondaires **C** et une portion de ladite partie dudit HF n'ayant pas réagi, avantageusement en bas de colonne de distillation ;
b2-2) distillation dudit courant **G1d** obtenu à l'étape b2-1) dans des conditions efficaces pour former un courant **G1d'** comprenant ladite portion de ladite partie des produits intermédiaires **B**, ladite portion de ladite partie dudit HF n'ayant pas réagi et une portion de ladite partie des produits secondaires **C** comprenant cis-1,3,3,3-tetrafluoro-1-propène (HFO-1234zeZ), 1,1,1,3,3,3-hexafluoropropane (HFC-236fa) et une partie de trans-1-chloro-3,3,3-trifluoro-1-propène (HCFO-1233zdE) et 1,1,1,3,3-pentafluoropropane (HFC-245fa) ; et un courant **G1d"** comprenant une portion de ladite partie des produits secondaires **C** comprenant cis-1-chloro-3,3,3-trifluoro-1-propène (HCFO-1233zdZ), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w = 0, w étant le nombre d'insaturations dans le composé de formule (II) considéré.

Selon un mode de réalisation préféré, la température en tête de colonne de distillation à l'étape b1) peut être de -10°C à -70°C, de préférence de -15°C à -65°C, en particulier de -20°C à -60°C, plus particulièrement de -25°C à -60°C, de manière privilégiée de -30°C à -60°C. De préférence, la pression en tête de colonne de distillation à l'étape b1) est de 2 à 20 bara, avantageusement de 3 à 15 bara, de préférence de 4 et 10 bara.

Selon un mode de réalisation préféré, la température en tête de colonne de distillation à l'étape b2-1) est comprise entre 0°C et 50°C. De préférence, la pression en tête de colonne de distillation à l'étape b2-1) est comprise entre 3 et 12 bara.

De préférence, la teneur en 2,3,3,3-tetrafluoropropène dans le courant **G1c** est supérieure à 40% en poids, avantageusement supérieure à 45% en poids, de préférence supérieure à 50% en poids, plus préférentiellement supérieure à 55% en poids, en particulier supérieure à 60% en poids, plus particulièrement supérieure à 65% en poids, de manière privilégiée supérieure à 70% en poids sur base du poids total du courant **G1c**.

De préférence, la teneur en produits intermédiaires **B** dans ledit courant **G1c** est comprise entre 5 et 40% en poids sur base du poids total dudit courant **G1c**, avantageusement entre 10 et 35% en poids sur base du poids total dudit courant **G1c**, de préférence entre 15 et 30% en poids sur base du poids total dudit courant **G1c**.

De préférence, la teneur en trans-1,3,3,3-tetrafluoropropène dans ledit courant **G1c** est inférieure à 15% en poids sur base du poids total dudit courant **G1c**, avantageusement inférieure à 12% en poids, de préférence inférieure à 10% en poids, plus préférentiellement inférieure à 8% en poids, en particulier inférieure à 6% en poids, plus particulièrement inférieure à 5% en poids sur base du poids total dudit courant **G1c**.

De préférence, la teneur en 2-chloro-3,3,3-trifluoropropène dans ledit courant **G1c** est inférieure à 0,5% en poids sur base du poids total dudit courant **G1c**, avantageusement inférieure à 0,1% en poids, de préférence inférieure à 500 ppm en poids, plus préférentiellement inférieure à 250 ppm en poids, en particulier inférieure à 100 ppm en poids, plus particulièrement inférieure à 50 ppm en poids sur base du poids total dudit courant **G1c**.

De préférence, la teneur en 1,1,1,2,2-pentafluoropropane dans ledit courant **G1c** est inférieure à 40% en poids sur base du poids total dudit courant **G1c**, avantageusement inférieure à 35% en poids, de préférence inférieure à 30% en poids, plus préférentiellement inférieure à 25% en poids, en particulier inférieure à 22% en poids, plus particulièrement inférieure à 20% en poids sur base du poids total dudit courant **G1c**.

De préférence, la teneur en acide fluorhydrique dans ledit courant **G1c** est inférieure à 20% en poids sur base du poids total dudit courant **G1c**, avantageusement inférieure à 18% en poids, de préférence inférieure à 16% en poids, plus préférentiellement inférieure à 14% en poids, en particulier inférieure à 12% en poids, plus particulièrement inférieure à 10% en poids sur base du poids total dudit courant **G1c**.

De préférence, la teneur en produits intermédiaires **B** dans ledit courant **G1d** est comprise entre 60 et 90% en poids sur base du poids total dudit courant **G1d,** avantageusement entre 70 et 90% en poids sur base du poids total dudit courant **G1d,** de préférence entre 75 et 85% en poids sur base du poids total dudit courant **G1d**.

De préférence, la teneur en trans-1,3,3,3-tetrafluoropropène dans ledit courant **G1d** est inférieure à 10% en poids sur base du poids total dudit courant **G1d**, avantageusement inférieure à 9% en poids, de préférence inférieure à 8% en poids, plus préférentiellement inférieure à 7% en poids, en particulier inférieure à 6% en poids, plus particulièrement inférieure à 5% en poids sur base du poids total dudit courant **G1d**.

De préférence, la teneur en 2-chloro-3,3,3-trifluoropropène dans ledit courant **G1d** est inférieure à 25% en poids sur base du poids total dudit courant **G1d,** avantageusement inférieure à 24% en poids, de préférence inférieure à 23% en poids, plus préférentiellement inférieure à 22% en poids, en particulier inférieure à 21% en poids, plus particulièrement inférieure à 20% en poids sur base du poids total dudit courant **G1d**.

De préférence, la teneur en 1,1,1,2,2-pentafluoropropane dans ledit courant **G1d** est inférieure à 75% en poids sur base du poids total dudit courant **G1d,** avantageusement inférieure à 74% en poids, de préférence inférieure à 73% en poids, plus préférentiellement inférieure à 72% en poids, en particulier inférieure à 71% en poids, plus particulièrement inférieure à 70% en poids sur base du poids total dudit courant **G1d**.

De préférence, la teneur en acide fluorhydrique dans ledit courant **G1d** est inférieure à 15% en poids sur base du poids total dudit courant **G1d,** avantageusement inférieure à 14% en poids, de préférence inférieure à 13% en poids, plus préférentiellement inférieure à 12% en poids sur base du poids total dudit courant **G1d**.

Selon un mode de réalisation préféré, la température en tête de colonne de distillation utilisée à l'étape b2-2) est de 0°C à 60°C, de préférence de 10°C à 45°C. En particulier, la pression en tête de colonne de distillation utilisée à l'étape b2-2) est de 1 à 10 bara, de préférence de 2 à 8 bara.

De préférence, le courant **G1d'** est sous forme liquide. De préférence le courant **G1d"** est sous forme liquide.

De préférence, au moins 90% du 1,1,1,2,2-pentafluoropropane contenu dans le courant **G1d** est récupéré dans le courant **G1d',** avantageusement au moins 91%, de préférence au moins 92%, plus préférentiellement au moins 93%, en particulier au moins 94%, plus particulièrement au moins 95%, de manière privilégiée au moins 96%, de manière préférentiellement privilégiée au moins 97%, de manière particulièrement privilégiée au moins 98% du 1,1,1,2,2-pentafluoropropane et du trans-1,3,3,3-tetrafluoropropène contenus dans le courant **G1d** sont récupérés dans le courant **G1d'**.

De préférence, au moins 90% du trans-1,3,3,3-tetrafluoropropène contenu dans le courant **G1d** est récupéré dans le courant **G1d',** avantageusement au moins 91%, de préférence au moins 92%, plus préférentiellement au moins 93%, en particulier au moins 94%, plus particulièrement au moins 95%, de manière privilégiée au moins 96%, de manière préférentiellement privilégiée au moins 97%, de manière particulièrement privilégiée au moins 98% du trans-1,3,3,3-tetrafluoropropène contenu dans le courant **G1d** est récupéré dans le courant **G1d'**.

De préférence, au moins 90% du 1,1,1,2,2-pentafluoropropane et du trans-1,3,3,3-tetrafluoropropène contenus dans le courant **G1d** sont récupérés dans le courant **G1d'**, avantageusement au moins 91%, de préférence au moins 92%, plus préférentiellement au moins 93%, en particulier au moins 94%, plus particulièrement au moins 95%, de manière privilégiée au moins 96%, de manière préférentiellement privilégiée au moins 97%, de manière particulièrement privilégiée au moins 98% du 1,1,1,2,2-pentafluoropropane et du trans-1,3,3,3-tetrafluoropropène contenus dans le courant **G1d** sont récupérés dans le courant **G1d'**.

De préférence, au moins 50% du 2-chloro-3,3,3-trifluoropropène contenu dans le courant **G1d** est récupéré dans le courant **G1d',** avantageusement au moins 55%, de préférence au moins 60%, plus préférentiellement au moins 65%, en particulier au moins 70%, plus particulièrement au moins 75% du 2-chloro-3,3,3-trifluoropropène contenu dans le courant **G1d** est récupéré dans le courant **G1d'**.

De préférence, le courant **G1d'** comprend de 60 à 70% en poids de 1,1,1,2,2-pentafluoropropane, de 1% à 5% en poids de trans-1,3,3,3-tetrafluoropropène et 10 à 15% en poids de 2-chloro-3,3,3-trifluoropropène sur base du poids total du courant **G1d'**.

De préférence, au moins 85% du cis-1,3,3,3-tetrafluoro-1-propène (HFO-1234zeZ) contenu dans le courant **G1d** est récupéré dans le courant **G1d',** avantageusement au moins 90%, de préférence au moins 92%, plus préférentiellement au moins 93%, en particulier au moins 95%, plus particulièrement au moins 97% du cis-1,3,3,3-tetrafluoro-1-propène (HFO-1234zeZ) contenu dans le courant **G1d** est récupéré dans le courant **G1d'**.

De préférence, au moins 85% du 1,1,1,3,3,3-hexafluoropropane (HFC-236fa) contenu dans le courant **G1d** est récupéré dans le courant **G1d',** avantageusement au moins 90%, de préférence au moins 92%, plus préférentiellement au moins 93%, en particulier au moins 95%, plus particulièrement au moins 97% du 1,1,1,3,3,3-hexafluoropropane (HFC-236fa) contenu dans le courant **G1d** est récupéré dans le courant **G1d'**.

De préférence, au moins 85% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d',** avantageusement au moins 90%, de préférence au moins 92%, plus préférentiellement au moins 93%, en particulier au moins 95%, plus particulièrement au moins 97% avantageusement au moins 90%, de préférence au moins 92%, plus préférentiellement au moins 93%, en particulier au moins 95%, plus particulièrement au moins 97%du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d'**.

De préférence, le courant **G1d'** comprend de 60 à 70% en poids de 1,1,1,2,2-pentafluoropropane, de 1% à 5% en poids de trans-1,3,3,3-tetrafluoropropène, de 10 à 15% en poids de 2-chloro-3,3,3-trifluoropropène, de 0,1 à 0,5% en poids de cis-1,3,3,3-tetrafluoro-1-propène (HFO-1234zeZ), de 1 à 5% en poids de 1,1,1,3,3,3-hexafluoropropane (HFC-236fa) sur base du poids total du courant **G1d'**.

De préférence, au moins 40% du trans-1-chloro-3,3,3-trifluoropropène (HCFO-1233zdE) contenu dans le courant **G1d** est récupéré dans le courant **G1d',** avantageusement au moins 50%, de préférence au moins 55%, plus préférentiellement au moins 60%, en particulier au moins 65%, plus particulièrement au moins 70% du trans-1-chloro-3,3,3-trifluoropropène (HCFO-1233zdE) contenu dans le courant **G1d** est récupéré dans le courant **G1d'**.

De préférence, au moins 40% du 1,1,1,3,3-pentafluoropropane (HFC-245fa) contenu dans le courant **G1d** est récupéré dans le courant **G1d',** avantageusement au moins 50%, de préférence au moins 55%, plus préférentiellement au moins 60%, en particulier au moins 65%, plus particulièrement au moins 70% du 1,1,1,3,3-pentafluoropropane (HFC-245fa) contenu dans le courant **G1d** est récupéré dans le courant **G1d'**.

De préférence, le courant **G1d'** comprend de 0,1 à 0,5% en poids de cis-1,3,3,3-tetrafluoro-1-propène (HFO-1234zeZ), de 1,0% à 5,0% en poids de 1,1,1,3,3,3-hexafluoropropane (HFC-236fa), 0,5 à 2,0% en poids de trans-1-chloro-3,3,3-trifluoro-1-propène (HCFO-1233zdE) et de 0,1 à 1,0% en poids de 1,1,1,3,3-pentafluoropropane (HFC-245fa) sur base du poids total du courant **G1d'**.

De préférence, le courant **G1d'** comprend de 60 à 70% en poids de 1,1,1,2,2-pentafluoropropane, de 1,0% à 5,0% en poids de trans-1,3,3,3-tetrafluoropropène, de 10 à 15% en poids de 2-chloro-3,3,3-trifluoropropène, de 0,1 à 0,5% en poids de cis-1,3,3,3-tetrafluoro-1-propène (HFO-1234zeZ), de 1,0 à 5,0% en poids de 1,1,1,3,3,3-hexafluoropropane (HFC-236fa), de 0,1 à 1,0% en poids de 1,1,1,3,3-pentafluoropropane (HFC-245fa) et de 0,5 à 2,0% en poids de trans-1-chloro-3,3,3-trifluoropropène (HCFO-1233zdE) sur base du poids total du courant **G1d'**.

De préférence, le courant **G1d'** comprend de 10 à 20% en poids d'acide fluorhydrique sur base du poids total du courant **G1d'**.

De préférence, le courant **G1d'** comprend également 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe) en une faible teneur. En particulier, la teneur en 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe) dans le courant **G1d'** est inférieure à 5% en poids sur base du poids total de **G1d',** avantageusement inférieure à 4% en poids, de préférence inférieure à 3% en poids, plus préférentiellement inférieure à 2% en poids, en particulier inférieure à 1% en poids, plus particulièrement inférieure à 0,5% en poids sur base du poids total de **G1d'**.

De préférence, ledit courant **G1d'** est dépourvu des autres produits secondaires **C** tels que décrits ci-dessus. Les autres produits secondaires **C** sont cis-1-chloro-3,3,3-trifluoro-1-propène (HCFO-1233zdZ) et un ou plusieurs composés de formule (II). Alternativement, ledit courant **G1d'** peut comprendre une très faible teneur en les autres produits secondaires **C** tels que décrits ci-dessus. De préférence, si le courant **G1d'** en contient, la teneur en chacun des autres produits secondaires **C** est inférieure à 1% en poids sur base du poids total dudit courant **G1d',** avantageusement inférieure à 0,5% en poids, de préférence inférieure à 0,1% en poids, plus préférentiellement inférieure à 0,05% en poids, en particulier inférieure à 0,01% en poids, plus particulièrement inférieure à 0,005% en poids sur base du poids total dudit courant **G1d'**. En particulier, si le courant **G1d'** en contient, la teneur total en cis-1-chloro-3,3,3-trifluoro-1-propène (HCFO-1233zdZ), et un ou plusieurs composés de formule (II) est inférieure à 1% en poids sur base du poids total dudit courant **G1d',** avantageusement inférieure à 0,5% en poids, de préférence inférieure à 0,1% en poids, plus préférentiellement inférieure à 0,05% en poids, en particulier inférieure à 0,01% en poids, plus particulièrement inférieure à 0,005% en poids sur base du poids total dudit courant **G1d'**.

De préférence, le courant **G1d'** est recyclé à l'étape a).

De préférence, au moins 95% du cis-1-chloro-3,3,3-trifluoro-1-propène (HCFO-1233zdZ) contenu dans le courant **G1d** est récupéré dans le courant **G1d",** avantageusement au moins 96%, de préférence au moins 97%, plus préférentiellement au moins 98%, en particulier au moins 99%, plus particulièrement au moins 99,5% du cis-1-chloro-3,3,3-trifluoro-1-propène (1233zdZ) contenu dans le courant **G1d** est récupéré dans le courant **G1d"**.

De préférence, au moins 95% dudit un ou plusieurs composés de formule (II) contenu dans le courant **G1d** est récupéré dans le courant **G1d",** avantageusement au moins 96%, de préférence au moins 97%, plus préférentiellement au moins 98%, en particulier au moins 99%, plus particulièrement au moins 99,5% dudit un ou plusieurs composés de formule (II) contenu dans le courant **G1d** est récupéré dans le courant **G1d"**.

De préférence, au moins 80% du 2-chloro-1,1,1,3,3-pentafluoropropane (HCFC-235da) contenu dans le courant **G1d** est récupéré dans le courant **G1d",** avantageusement au moins 85%, de préférence au moins 90%, plus préférentiellement au moins 93%, en particulier au moins 94%, plus particulièrement au moins 95% du 2-chloro-1,1,1,3,3-pentafluoropropane (HCFC-235da) contenu dans le courant **G1d** est récupéré dans le courant **G1d"**. En particulier, de 90% à 98% du 2-chloro-1,1,1,3,3-pentafluoropropane (HCFC-235da) contenu dans le courant **G1d** est récupéré dans le courant **G1d"**.

De préférence, au moins 80% du 1,2-dichloro-3,3,3-trifluoropropène (HCFO-1223xd) contenu dans le courant **G1d** est récupéré dans le courant **G1d",** avantageusement au moins 85%, de préférence au moins 90%, plus préférentiellement au moins 93%, en particulier au moins 94%, plus particulièrement au moins 95% du 1,2-dichloro-3,3,3-trifluoropropène (HCFO-1223xd) contenu dans le courant **G1d** est récupéré dans le courant **G1d"**. En particulier, de 90% à 98% du 1,2-dichloro-3,3,3-trifluoropropène (HCFO-1223xd) contenu dans le courant **G1d** est récupéré dans le courant **G1d"**.

De préférence, au moins 80% du 2-chloro-1,3,3,3-tetrafluoropropène (HCFO-1224xe) contenu dans le courant **G1d** est récupéré dans le courant **G1d",** avantageusement au moins 85%, de préférence au moins 90%, plus préférentiellement au moins 93%, en particulier au moins 94%, plus particulièrement au moins 95% du 2-chloro-1,3,3,3-tetrafluoropropène (HCFO-1224xe) contenu dans le courant **G1d** est récupéré dans le courant **G1d"**. En particulier, de 90% à 98% du 2-chloro-1,3,3,3-tetrafluoropropène (HCFO-1224xe) contenu dans le courant **G1d** est récupéré dans le courant **G1d"**.

De préférence, ledit courant **G1d"** comprend de 1 à 25% en poids de 2-chloro-1,3,3,3-tetrafluoropropène (HCFO-1224xe), avantageusement de 5 à 20% en poids, de préférence de 7 à 18% en poids, en particulier de 10 à 15% en poids de 2-chloro-1,3,3,3-tetrafluoropropène (HCFO-1224xe) sur base du poids total dudit courant **G1d"**.

De préférence, ledit courant **G1d"** comprend de 40 à 85% en poids de 1,2-dichloro-3,3,3-trifluoropropène (HCFO-1223xd), avantageusement de 45 à 80% en poids, de préférence de 50 à 75% en poids, en particulier de 55 à 70% en poids de 1,2-dichloro-3,3,3-trifluoropropène (HCFO-1223xd) sur base du poids total dudit courant **G1d"**.

De préférence, ledit courant **G1d"** comprend de 1 à 25% en poids de 2-chloro-1,1,1,3,3-pentafluoropropane (HCFC-235da), avantageusement de 5 à 20% en poids, de préférence de 7 à 18% en poids, en particulier de 10 à 15% en poids de 2-chloro-1,1,1,3,3-pentafluoropropane (HCFC-235da) sur base du poids total dudit courant **G1d"**.

De préférence, ledit courant **G1d"** comprend de 0,05 à 10% en poids de cis-1-chloro-3,3,3-trifluoro-1-propène (HCFO-1233zdZ), avantageusement de 0,1 à 8% en poids, de préférence de 0,5 à 7% en poids, en particulier de 1 à 5% en poids de cis-1-chloro-3,3,3-trifluoro-1-propène (HCFO-1233zdZ) sur base du poids total dudit courant **G1d"**.

De préférence, ledit courant **G1d"** comprend de 0.1 à 10% en poids de un ou plusieurs composés de formule (II), avantageusement de 0,25 à 7,5% en poids, de préférence de 0,5 à 5% en poids, en particulier de 1 à 5% en poids de un ou plusieurs composés de formule (II) sur base du poids total dudit courant **G1d"**.

Ainsi, ledit courant **G1d"** peut comprendre de 1 à 5% en poids de cis-1-chloro-3,3,3-trifluoro-1-propène (HCFO-1233zdZ), de 10% à 15% en poids de 2-chloro-1,1,1,3,3-pentafluoropropane (HCFC-235da), de 55 à 70% en poids de 1,2-dichloro-3,3,3-trifluoropropène (HCFO-1223xd), de 10 à 15% en poids de 2-chloro-1,3,3,3-tetrafluoropropène (HCFO-1224xe), de 1 à 5% en poids dudit un ou plusieurs composés de formule (II) sur base du poids total du courant **G1d"**.

De préférence, au plus 15% du cis-1,3,3,3-tetrafluoro-1-propène (HFO-1234zeZ) contenu dans le courant **G1d** est récupéré dans le courant **G1d",** avantageusement au plus 10%, de préférence au plus 8%, plus préférentiellement au plus 7%, en particulier au plus 5%, plus particulièrement au plus 3% du cis-1,3,3,3-tetrafluoro-1-propène (HFO-1234zeZ) contenu dans le courant **G1d** est récupéré dans le courant **G1d"**.

De préférence, au plus 15% du 1,1,1,3,3,3-hexafluoropropane (HFC-236fa) contenu dans le courant **G1d** est récupéré dans le courant **G1d",** avantageusement au plus 10%, de préférence au plus 8%, plus préférentiellement au plus 7%, en particulier au plus 5%, plus particulièrement au plus 3% du 1,1,1,3,3,3-hexafluoropropane (HFC-236fa) contenu dans le courant **G1d** est récupéré dans le courant **G1d"**.

De préférence, au plus 15% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d',** avantageusement au plus 10%, de préférence au plus 8%, plus préférentiellement au plus 7%, en particulier au plus 5%, plus particulièrement au plus 3% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d"**.

De préférence, au plus 60% du trans-1-chloro-3,3,3-trifluoropropène (HCFO-1233zdE) contenu dans le courant **G1d** est récupéré dans le courant **G1d",** avantageusement au plus 50%, de préférence au plus 45%, plus préférentiellement au plus 40%, en particulier au plus 35%, plus particulièrement au plus 30% du trans-1-chloro-3,3,3-trifluoropropène (HCFO-1233zdE) contenu dans le courant **G1d** est récupéré dans le courant **G1d"**.

De préférence, au plus 60% du 1,1,1,3,3-pentafluoropropane (HFC-245fa) contenu dans le courant **G1d** est récupéré dans le courant **G1d"**, avantageusement au plus 50%, de préférence au plus 45%, plus préférentiellement au plus 40%, en particulier au plus 35%, plus particulièrement au plus 30% du 1,1,1,3,3-pentafluoropropane (HFC-245fa) contenu dans le courant **G1d** est récupéré dans le courant **G1d"**.

De préférence, le courant **G1d"** comprend de 0,01 à 0,05% en poids de cis-1,3,3,3-tetrafluoro-1-propène (HFO-1234zeZ), de 0,05% à 0,5% en poids de 1,1,1,3,3,3-hexafluoropropane (HFC-236fa), 0,05 à 0,5% en poids de trans-1-chloro-3,3,3-trifluoro-1-propène (HCFO-1233zdE) et de 0,05 à 0,5% en poids de 1,1,1,3,3-pentafluoropropane (HFC-245fa) sur base du poids total du courant **G1d"**.

De préférence, le courant **G1d"** comprend moins de 2% en poids d'acide fluorhydrique sur base du poids total du courant **G1d"**, avantageusement moins de 1% en poids, de préférence moins de 0,5% en poids, en particulier moins de 0,1% en poids d'acide fluorhydrique sur base du poids total du courant **G1d"**.

De préférence, au plus 50% du 2-chloro-3,3,3-trifluoropropène contenu dans le courant **G1d** est récupéré dans le courant **G1d"**, avantageusement au plus 45%, de préférence au plus 40%, plus préférentiellement au plus 35%, en particulier au plus 30%, plus particulièrement au plus 25% du 2-chloro-3,3,3-trifluoropropène contenu dans le courant **G1d** est récupéré dans le courant **G1d"**.

De préférence, le courant **G1d"** comprend de 1 à 25% en poids de 2-chloro-3,3,3-trifluoropropène sur base du poids total du courant **G1d"**, avantageusement de 5 à 20% en poids, de préférence de 7 à 18% en poids, en particulier de 10 à 15% en poids de 2-chloro-3,3,3-trifluoropropène sur base du poids total du courant **G1d"**.

De préférence, le procédé comprend également les étapes :
b3) mise en contact du courant **G1c** obtenu à l'étape b2-1) avec une solution aqueuse d'acide fluorhydrique de concentration supérieure à 40% pour former un courant **G1c'** diphasique comprenant 2,3,3,3-tétrafluoropropène (HFO-1234yf), de l'acide fluorhydrique, une portion de ladite partie des produits intermédiaires **B** et une portion de ladite partie des produits secondaires **C**,
b4) stockage dudit courant **G1c'** dans un réservoir tampon, ledit courant **G1c'** étant un courant diphasique constitué d'une phase liquide et d'une phase gazeuse,
b5) passage de la phase gazeuse dudit courant **G1c'** dans une colonne d'absorption alimenté à contre-courant par un flux aqueux pour former un courant **G1c"** comprenant 2,3,3,3-tétrafluoropropène (HFO-1234yf), une portion de ladite partie des produits intermédiaires **B** et une portion de ladite partie des produits secondaires **C** et un courant **G1c'''** comprenant HF.

De préférence, le courant **G1c** formé à l'étape b2-1) peut comprendre 2,3,3,3-tétrafluoropropène (HFO-1234yf), 1,1,1,2,2-pentafluoropropane (HFC-245cb) et 1,3,3,3-tetrafluoro-1-propène (HFO-1234ze).

De préférence, la solution aqueuse d'acide fluorhydrique utilisée à l'étape b3) est de concentration supérieure à 40% en poids. En particulier, la solution aqueuse d'acide fluorhydrique est de concentration supérieure à 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94% ou 95% en poids. Plus particulièrement, la solution aqueuse d'acide fluorhydrique est de concentration supérieure ou égale à 50% en poids, ou supérieure ou égale 60% en poids ou supérieure ou égale à 70% en poids.

Plus particulièrement, la solution aqueuse d'acide fluorhydrique utilisée à l'étape b3) peut être comprise entre l'une quelconque des valeurs mentionnées ci-dessus. Ainsi, la solution aqueuse d'acide fluorhydrique peut être comprise entre 45% et 95% en poids, entre 50% et 90% en poids, entre 55% et 85% en poids, entre 60% en poids et 80% en poids ou entre 65% en poids et 75% en poids.

Selon un mode de réalisation préféré le procédé comprend également les étapes :
b6) neutralisation dudit courant **G1c"** obtenu à l'étape b5) par une solution aqueuse alcaline pour former un courant neutralisé, et
b7) séchage dudit courant neutralisé obtenu à l'étape b6) sur tamis moléculaire pour former un courant neutralisé et séché **G1c""**.

De préférence, la solution aqueuse d'acide fluorhydrique utilisé à l'étape b3) est à une température comprise entre -20°C à 80°C avant sa mise en contact avec le courant **G1c**, avantageusement entre -15°C et 70°C, de préférence entre -10°C et 60°C, plus préférentiellement entre -5°C et 50°C, en particulier entre -5°C et 40°C, plus particulièrement entre 0°C et 30°C. Ainsi, dans un mode de réalisation particulièrement préféré, la température de la solution aqueuse d'acide fluorhydrique utilisé à l'étape b3), avant sa mise en contact avec le courant **G1c**, peut être de 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C ou 30°C. La mise en œuvre de ladite solution aqueuse d'acide fluorhydrique aux températures mentionnées ci-dessus a pour but de contrôler l'exothermicité survenant lors de la mise en contact de celle-ci avec le courant **G1c**.

Selon un mode de réalisation préféré, ledit courant **G1c'** diphasique consiste en une phase gazeuse comprenant 2,3,3,3-tétrafluoropropène (HFO-1234yf), une portion de ladite partie des produits intermédiaires **B** et une portion de ladite partie des produits secondaires **C**. La phase gazeuse peut éventuellement comprendre des traces d'acide fluorhydrique. Dans ce cas, la teneur en acide fluorhydrique dans ladite phase gazeuse dudit courant diphasique est inférieure à 5% en poids sur base du poids total de ladite phase gazeuse, en particulier inférieure à 2% en poids sur base du poids total de ladite phase gazeuse, plus particulièrement inférieure à 1% en poids sur base du poids total de ladite phase gazeuse. La phase liquide dudit courant diphasique comprend de l'acide fluorhydrique. La phase liquide dudit courant diphasique **G1c'** peut également comprendre moins de 5% en poids sur base du poids total de ladite phase liquide de composés organiques sélectionnés parmi le groupe consistant en 2,3,3,3-tétrafluoropropène (HFO-1234yf), une portion de ladite partie des produits intermédiaires **B** et une portion de ladite partie des produits secondaires **C ;** de préférence moins de 1% en poids, en particulier moins de 5000 ppm, plus particulièrement moins de 1000 ppm, de manière privilégiée moins de 500 ppm, de manière particulièrement privilégiée moins de 100 ppm de composés organiques sélectionnés parmi le groupe consistant en 2,3,3,3-tétrafluoropropène (HFO-1234yf), une portion de ladite partie des produits intermédiaires **B** et une portion de ladite partie des produits secondaires **C** sur base du poids total de ladite phase liquide.

De préférence, la concentration en acide fluorhydrique dans ladite phase liquide dudit courant diphasique **G1c'** est supérieure à la concentration de ladite solution aqueuse d'acide fluorhydrique utilisée à l'étape b3). Ladite phase liquide dudit courant diphasique **G1c'** peut avoir une concentration en acide fluorhydrique supérieure à 41% en poids sur base du poids total de ladite phase liquide dudit courant diphasique **G1c'**. Avantageusement, ladite phase liquide dudit courant diphasique **G1c'** peut avoir une concentration en acide fluorhydrique supérieure à 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94% ou 95% en poids sur base du poids total de ladite phase liquide dudit courant diphasique **G1c'**. De préférence, ladite phase liquide dudit courant diphasique **G1c'** peut avoir une concentration en acide fluorhydrique comprise entre 45% et 95% en poids, entre 50% et 90% en poids, entre 55% et 85% en poids, entre 60% en poids et 80% en poids ou entre 65% en poids et 75% en poids tout en étant supérieure à la concentration de ladite solution aqueuse d'acide fluorhydrique utilisée à l'étape b3).

Comme mentionné ci-dessus, l'étape b4) du procédé selon la présente invention met en œuvre le stockage dudit courant diphasique **G1c'** dans un réservoir tampon, ledit courant diphasique **G1c'** étant constitué de ladite phase liquide et de ladite phase gazeuse telle que décrit ci-dessus.

Comme mentionné ci-dessus, l'étape b5) du procédé selon la présente invention met en œuvre, le passage de la phase gazeuse dudit courant diphasique **G1c'** dans une colonne d'absorption alimenté à contre-courant par un flux aqueux pour former un courant **G1c"** comprenant 2,3,3,3-tétrafluoropropène (HFO-1234yf), une portion de ladite partie des produits intermédiaires **B** et une portion de ladite partie des produits secondaires **C** et un courant **G1c'''** comprenant l'acide fluorhydrique.

De préférence, le débit du flux aqueux utilisé à l'étape b5) est déterminé en fonction de la quantité d'acide fluorhydrique contenue dans ledit courant **G1c**. Ainsi, le rapport entre le débit du flux aqueux exprimé en kg/h alimentant la colonne d'absorption à l'étape b5) et la quantité d'acide fluorhydrique dans ledit courant **G1c** exprimée en kg/h est compris entre 0,05 et 1,22. Avantageusement, le rapport entre le débit du flux aqueux exprimé en kg/h alimentant la colonne d'absorption à l'étape b5) et la quantité d'acide fluorhydrique dans ledit courant **G1c** exprimée en kg/h peut être compris de 0,11 à 1,00, de préférence de 0,18 à 0,82, plus préférentiellement de 0,25 à 0,67, en particulier de 0,33 à 0,54. Ainsi le rapport entre le débit du flux aqueux exprimé en kg/h alimentant la colonne d'absorption à l'étape b5) et la quantité d'acide fluorhydrique dans ledit courant **G1c** exprimée en kg/h peut être de 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69 ou 0,70. Un courant aqueux supplémentaire correspondant à la fraction d'eau vaporisée en tête de ladite colonne d'absorption peut également alimenter ladite colonne. Le flux aqueux tel que décrit ci-dessus est différent dudit courant aqueux supplémentaire lié à la fraction d'eau vaporisée en tête de la colonne et ne l'englobe pas.

Selon un mode de réalisation préféré, ladite colonne d'absorption mise en œuvre à l'étape b5) comprend au moins un étage d'absorption, avantageusement deux étages d'absorption. De préférence, ladite colonne d'absorption mise en œuvre à l'étape b5) comprend au moins trois étages d'absorption. Ladite colonne d'absorption mise en œuvre à l'étape b5) peut ainsi comprendre deux, trois, quatre, cinq, six, sept, huit, neuf, dix, onze, douze, treize, quatorze ou quinze étages d'absorption.

La mise en œuvre d'une colonne d'absorption ayant au moins un étage, avantageusement au moins deux étages d'absorption, de préférence au moins trois étages d'absorption, permet d'obtenir un courant **G1c"** ayant une faible teneur en acide fluorhydrique. Avantageusement, ledit courant **G1c"** comprend moins de 1000 ppm d'acide fluorhydrique en poids sur base du poids total dudit courant **G1c"**, de préférence moins de 900 ppm d'acide fluorhydrique, plus préférentiellement moins de 800 ppm d'acide fluorhydrique, en particulier moins de 700 ppm d'acide fluorhydrique, plus particulièrement moins de 600 ppm d'acide fluorhydrique, de manière privilégiée moins de 500 ppm d'acide fluorhydrique, de manière encore plus privilégiée moins de 400 ppm d'acide fluorhydrique, de manière préférentiellement privilégiée moins de 300 ppm d'acide fluorhydrique, de manière particulièrement privilégiée moins de 200 ppm d'acide fluorhydrique, de manière plus particulièrement privilégiée moins de 100 ppm d'acide fluorhydrique. Ainsi, ledit courant **G1c"** peut avoir une teneur en acide fluorhydrique comprise entre 1 et 200 ppm, entre 5 et 190 ppm, entre 10 et 180 ppm, entre 15 et 170 ppm, entre 20 et 160 ppm, entre 25 et 150 ppm ou entre 30 et 140 ppm en poids sur base du poids total dudit courant **G1c"**. Ledit courant **G1c"** peut avoir une teneur en acide fluorhydrique inférieure à 100 ppm, avantageusement inférieure à 75 ppm, de préférence inférieure à 50 ppm, plus préférentiellement inférieure à 30 ppm, en particulier inférieure à 15 ppm, plus particulièrement inférieure à 10 ppm en poids sur base du poids total dudit courant **G1c"**.

De préférence, au moins 80% en poids de l'acide fluorhydrique éventuellement présent dans ladite phase gazeuse dudit courant diphasique **G1c'** est absorbé par le premier étage d'absorption de ladite colonne d'absorption, en particulier au moins 85% en poids de l'acide fluorhydrique éventuellement présent dans ladite phase gazeuse dudit courant diphasique **G1c'** est absorbé par le premier étage d'absorption de ladite colonne d'absorption, plus particulièrement au moins 90% en poids de l'acide fluorhydrique éventuellement présent dans ladite phase gazeuse dudit courant diphasique **G1c'** est absorbé par le premier étage d'absorption de ladite colonne d'absorption.

De préférence, ledit flux aqueux peut être introduit au moins au niveau de la tête de la colonne d'absorption. De préférence, la température en tête de ladite colonne d'absorption est de 20°C à 70°C, de préférence de 30°C à 50°C.

Selon un mode de réalisation préféré, ledit courant **G1c'''** est sous la forme d'une solution aqueuse d'acide fluorhydrique. Avantageusement, ledit courant **G1c'''** est une solution d'acide fluorhydrique de concentration inférieure à 30% en poids sur base du poids total dudit courant **G1c'''**. De préférence, ledit courant **G1c'''** est une solution d'acide fluorhydrique de concentration inférieure à 25% en poids sur base du poids total dudit courant **G1c'''**. En particulier, ledit courant **G1c'''** est une solution d'acide fluorhydrique de concentration comprise entre 5 et 25% en poids sur base du poids total dudit courant **G1c'''**, plus particulièrement, entre 10 et 20% en poids sur base du poids total dudit courant **G1c'''**. Selon un mode de réalisation préféré, ledit courant **G1c'''** est recyclé à l'étape b4). Le courant **G1c'''** est ainsi mélangé à la phase liquide dudit courant **G1c'**.

Selon un mode de réalisation préféré, ledit procédé comprend également les étapes de :
b6) neutralisation dudit courant **G1c"** obtenu à l'étape b5) par une solution aqueuse alcaline pour former un courant neutralisé, et
b7) séchage dudit courant neutralisé obtenu à l'étape b6), de préférence, sur tamis moléculaire pour former un courant neutralisé et séché **G1c""**.

Selon un mode de réalisation préféré, ladite solution aqueuse alcaline peut être une solution aqueuse d'hydroxyde d'un métal alcalin ou alcalino-terreux. La solution aqueuse alcaline peut être une solution aqueuse d'hydroxyde de sodium, d'hydroxyde de potassium, d'hydroxyde de calcium ou d'hydroxyde de magnésium ou un mélange de ceux-ci. De préférence, ladite solution aqueuse alcaline présente une concentration comprise entre 5 et 50% en poids sur base du poids total de ladite solution aqueuse alcaline. Avantageusement, ladite solution aqueuse alcaline présente une concentration d'au moins 5%, d'au moins 6%, d'au moins 7%, d'au moins 8%, d'au moins 9%, d'au moins 10%, d'au moins 11%, d'au moins 12%, d'au moins 13%, d'au moins 14%, d'au moins 15%, d'au moins 16% ou d'au moins 17% en poids sur base du poids total de ladite solution aqueuse alcaline ; et d'au plus 50%, d'au plus 48%, d'au plus 46%, d'au plus 44%, d'au plus 42%, d'au plus 40%, d'au plus 38%, d'au plus 36%, d'au plus 34%, d'au plus 32%, d'au plus 30%, d'au plus 28%, d'au plus 26%, d'au plus 24%, d'au plus 22% en poids sur base du poids total de ladite solution aqueuse alcaline.

Ledit courant neutralisé formé à l'étape b6) comprend de préférence 2,3,3,3-tétrafluoropropène (HFO-1234yf), une portion de ladite partie des produits intermédiaires **B** et une portion de ladite partie des produits secondaires **C**. De préférence, ledit courant neutralisé formé à l'étape b6) comprend de préférence 2,3,3,3-tétrafluoropropène (HFO-1234yf), 1,1,1,2,2-pentafluoropropane (HFC-245cb) et trans-1,3,3,3-tetrafluoro-1-propène (HFO-1234zeE).

La teneur en acide fluorhydrique dans ledit courant neutralisé est inférieure à la teneur en acide fluorhydrique dudit courant **G1c"**, avant sa neutralisation. Ledit courant neutralisé formé à l'étape b6) peut également contenir de l'eau.

Ledit courant neutralisé formé à l'étape b6) peut ainsi être séché à l'étape b7) du présent procédé. De préférence, ledit courant neutralisé formé à l'étape b6) est séché sur tamis moléculaire. Par exemple, ledit courant neutralisé formé à l'étape b6) est séché sur tamis moléculaire de 3A, tel que la siliporite.

L'étape b7) du présent procédé permet la formation d'un courant neutralisé et séché **G1c""** comprenant 2,3,3,3-tétrafluoropropène (HFO-1234yf), une portion de ladite partie des produits intermédiaires **B** et une portion de ladite partie des produits secondaires **C**. Ledit courant **G1c""** peut éventuellement être comprimé et liquéfié à une pression d'au plus 8 bara pour former un courant comprimé dans lequel 2,3,3,3-tétrafluoropropène (HFO-1234yf), une portion de ladite partie des produits intermédiaires **B** et une portion de ladite partie des produits secondaires **C** sont sous forme liquide.

Selon un mode de réalisation préféré, la phase liquide résultant du mélange de ladite phase liquide du courant **G1c'** et du courant **G1c'''** est recyclée à l'étape b3). La phase liquide résultant du mélange de ladite phase liquide du courant **G1c'** et du courant **G1c'''** peut avoir une concentration en acide fluorhydrique supérieure à 41% en poids sur base du poids total de ladite phase liquide résultant du mélange de ladite phase liquide du courant **G1c'** et du courant **G1c'''**. Avantageusement, ladite phase liquide résultant du mélange de ladite phase liquide du courant **G1c'** et du courant **G1c'''** peut avoir une concentration en acide fluorhydrique supérieure à 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94% ou 95% en poids sur base du poids total de ladite phase liquide résultant du mélange de ladite phase liquide du courant **G1c'** et du courant **G1c'''**. De préférence, ladite phase liquide résultant du mélange de ladite phase liquide du courant **G1c'** et du courant **G1c'''** peut avoir une concentration en acide fluorhydrique comprise entre 45% et 95% en poids, entre 50% et 90% en poids, entre 55% et 85% en poids, entre 60% en poids et 80% en poids ou entre 65% en poids et 75% en poids sur base du poids total de ladite phase liquide résultant du mélange de ladite phase liquide du courant **G1c'** et du courant **G1c'''**.

Selon un autre mode de réalisation préféré, la phase liquide résultant du mélange de ladite phase liquide du courant **G1c'** et du courant **G1c'''** est distillée pour former un courant **G2c**, de préférence en tête de colonne de distillation. Avantageusement, ledit courant **G2c** comprend de l'acide fluorhydrique contenant moins de 3000 ppm d'eau, de préférence moins de 2000 ppm d'eau, plus préférentiellement moins de 1000 ppm d'eau, en particulier moins de 500 ppm d'eau, plus particulièrement moins de 200 ppm d'eau, de manière privilégiée moins de 100 ppm d'eau, de manière plus privilégiée moins de 50 ppm d'eau sur base du poids total du courant **G2c**. Ledit courant **G2c** peut comprendre également moins de 50 ppm d'acide chlorhydrique, avantageusement moins de 45 ppm d'acide chlorhydrique, de préférence moins de 40 ppm d'acide chlorhydrique, plus préférentiellement moins de 35 ppm d'acide chlorhydrique, en particulier moins de 30 ppm d'acide chlorhydrique, plus particulièrement moins de 20 ppm d'acide chlorhydrique sur base du poids total du courant **G2c**. Ledit courant **G2c** peut comprendre également moins de 50 ppm de composés organiques, avantageusement moins de 45 ppm de composés organiques, de préférence moins de 40 ppm de composés organiques, plus préférentiellement moins de 35 ppm de composés organiques, en particulier moins de 30 ppm de composés organiques, plus particulièrement moins de 20 ppm de composés organiques sur base du poids total du courant **G2c**. Un composé organique est un composé comprenant au moins un atome de carbone. La température en tête de colonne de distillation peut être à une température de 10°C à 60°C, de préférence de 20°C à 50°C.

En outre, la distillation de ladite phase liquide résultant du mélange de ladite phase liquide du courant **G1c'** et du courant **G1c'''** forme un courant **G3c**, de préférence en pied de colonne de distillation, comprenant de l'acide fluorhydrique sous forme d'une solution aqueuse de concentration inférieure à 50% en poids. Avantageusement, ledit courant **G3c** comprenant de l'acide fluorhydrique sous forme d'une solution aqueuse de concentration inférieure à 50% en poids, 49% en poids, 48% en poids, 47% en poids, 46% en poids, 45% en poids, 44% en poids, 43% en poids, 42% en poids sur base du poids total dudit courant **G3c**. De préférence, ledit courant **G3c** comprenant de l'acide fluorhydrique sous forme d'une solution aqueuse de concentration supérieure à 20% en poids sur base du poids total dudit courant **G3c**. En particulier, ledit courant **G3c** comprenant de l'acide fluorhydrique sous forme d'une solution aqueuse de concentration supérieure 21% en poids, 22% en poids, 23% en poids, 24% en poids, 25% en poids, 26% en poids, 27% en poids, 28% en poids, 29% en poids, 30% en poids, 31% en poids, 32% en poids, 33% en poids, 34% en poids, 35% en poids sur base du poids total dudit courant **G3c.** Ladite solution aqueuse obtenue dans le courant **G3c** peut être commercialisé ou détruite par neutralisation.

Selon un mode de réalisation préféré, le procédé comprend une étape c), subséquente à l'étape b5) ou à l'étape b7), dans laquelle le courant **G1c"** obtenu à l'étape b5) ou le courant **G1c""** obtenu à l'étape b7) comprend 2,3,3,3-tétrafluoropropène (HFO-1234yf), 1,1,1,2,2-pentafluoropropane (HFC-245cb) et trans-1,3,3,3-tetrafluoro-1-propène (HFO-1234zeE) ; et ledit courant **G1c" ou G1c""** est distillé pour former un courant **G1e** comprenant 2,3,3,3-tétrafluoropropène (HFO-1234yf) et un courant **G1f** comprenant 1,1,1,2,2-pentafluoropropane (HFC-245cb) et trans-1,3,3,3-tetrafluoro-1-propène (HFO-1234zeE).

Le courant **G1f** obtenu à l'étape c) peut être séparé par distillation extractive.

Selon un mode de réalisation particulier, le courant **G1f** obtenu à l'étape c) est séparé par distillation extractive suivant les étapes :
c1) mise en contact dudit courant **G1f** obtenu à l'étape c) avec un agent d'extraction organique pour former un courant **G1g**, et
c2) distillation extractive du courant **G1g** pour former un flux **G1h** comprenant 1,1,1,2,2-pentafluoropropane (HFC-245cb), avantageusement en tête de colonne de distillation, et une composition **G1i** comprenant trans-1,3,3,3-tetrafluoro-1-propène (HFO-1234zeE) et ledit agent d'extraction organique, avantageusement en bas de colonne de distillation.

De préférence, le courant **G1i** comprenant trans-1,3,3,3-tetrafluoro-1-propène (HFO-1234zeE) et ledit agent d'extraction organique est séparé par distillation pour former un courant **G1j** comprenant ledit agent d'extraction organique et un courant **G1k** comprenant trans-1,3,3,3-tetrafluoro-1-propène (HFO-1234zeE). Le courant **G1j** comprenant ledit agent d'extraction organique peut être recyclé à l'étape c1). Le courant **G1k** comprenant trans-1,3,3,3-tetrafluoro-1-propène (HFO-1234zeE) peut être soit purifié ou détruit par incinération.

Selon un mode de réalisation préféré, ledit agent d'extraction organique est un solvant choisi parmi le groupe consistant en hydrohalocarbure, alcool, cétone, amine, ester, éther, aldéhyde, nitrile, carbonate, thioalkyle, amide, hétérocycle. Avantageusement, ledit agent d'extraction organique est un solvant sélectionné parmi le groupe consistant en alcool, cétone, amine, ester et hétérocycle. Selon un mode de réalisation préféré, ledit agent d'extraction organique a un point d'ébullition compris entre 10 et 150°C.

De préférence, ledit agent d'extraction peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle
γ_{1,S} représente le coefficient d'activité du 1,1,1,2,2-pentafluoropropane dans ledit agent d'extraction organique à dilution infinie,
P1 représente la pression de vapeur saturante du 1,1,1,2,2-pentafluoropropane,
γ_{2,S} représente le coefficient d'activité dudit trans-1,3,3,3-tetrafluoro-1-propène (HFO-1234zeE) dans ledit agent d'extraction organique à dilution infinie,
P2 représente la pression de vapeur saturante dudit trans-1,3,3,3-tetrafluoro-1-propène (HFO-1234zeE) ;
avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

La pression de vapeur saturante est considérée pour une température de 25°C.

De préférence, ledit agent d'extraction organique peut avoir une capacité de séparation C_{2,S} supérieure ou égale à 0,20, ladite capacité de séparation étant calculée par la formule C_{2,S} = 1/(γ_{2,S}) dans laquelle γ₂,_{S} représente le coefficient d'activité dudit trans-1,3,3,3-tetrafluoro-1-propène (HFO-1234zeE) dans ledit agent d'extraction organique à dilution infinie ; avantageusement, la capacité de séparation C_{2,S} est supérieure ou égale à 0,40, de préférence supérieure ou égale à 0,60, plus préférentiellement supérieure ou égale à 0,80, en particulier supérieure ou égale à 1,0.

De préférence, ledit agent d'extraction organique peut avoir un facteur de séparation S_{1,2} supérieur ou égal à 1,5 et une capacité d'absorption C_{2,S} supérieure ou égale à 0,6 et être sélectionné parmi le groupe consistant en éthylamine, acetaldehyde, isopropylamine, methylformate, diethylether, 1,2-epoxypropane, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, isopropylformate, diisopropylether, 2-ethoxy-2-methyl-propane, 1-butylamine, ethylacetate, butanone, n-propylformate, 2-ethoxy-butane, l-methoxy-2-methyl-butane, 2,2-dimethoxypropane, 1-ethoxy-2-methylpropane, diisopropylamine, 1,2-dimethoxyethane, 3-methyl-2-butanamine, diethoxymethane, isopropylacetate, di-n-propylether, 3-pentylamine, n-methylbutylamine, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 1,2-dimethoxypropane, 1,4-dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, dipropylamine, 2-ethoxyethanamine, sec-butylacetate, n-methyl-1,2-ethanediamine, 2,2-diethoxypropane, pyridine, 4-methyl-2-pentanone, 1,2-diaminoethane, butyronitrile, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol. Avantageusement, ledit agent d'extraction organique peut avoir un facteur de séparation S_{1,2} supérieur ou égal à 1,8 et/ou une capacité d'absorption C_{2,S} supérieure ou égale à 0,8 ; et être sélectionné parmi le groupe consistant en éthylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, 1,4-dioxane, 3-pentanone, 2-pentanone, 2-methoxy-1propanamine, trimethoxymethane, n-pentylamine, 3,3-dimethyl-2-butanone, 1,3-dioxane, piperidine, 2-ethoxyethanamine, n-methyl-1,2-ethanediamine, 1,2-diaminoethane, butyronitrile, 1-methoxy2-propanol, 1,2-propanediamine, 1-(dimethylamino)-2-propanol, 2-ethylbutylamine, diethylcarbonate, n-butylacetate, 2-hexanone, n-ethylethylenediamine, 2-methoxy1-propanol, 1, ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, 2-ethoxy-1-propanol, 1-methylpiperazine, 1,3-propanediamine, valeronitrile, 2-heptanamine, n,n-diethylethylenediamine, 4-methyl-2-hexanone, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol. De préférence, ledit agent d'extraction organique peut avoir un facteur de séparation S_{1,2} supérieur ou égal à 1,9 et/ou une capacité d'absorption C_{2,S} supérieure ou égale à 0,9 et être sélectionné parmi le groupe consistant en éthylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, 1,4-dioxane, 3-pentanone, 2-pentanone, 2-methoxy-1propanamine, n-pentylamine, 3,3-dimethyl-2-butanone, 1,3-dioxane, piperidine, 2-ethoxyethanamine, n-methyl-1,2-ethanediamine, 1,2-diaminoethane, 1,2-propanediamine, 1-(dimethylamino)-2-propanol, 2-ethylbutylamine, n-butylacetate, 2-hexanone, n-ethylethylenediamine, 1-ethoxy-2-propanol, 4-methyl-2-hexanamine, hexylamine, 2-(dimethylamino)-ethanol, cyclohexylamine, n-ethyl-2-dimethylaminoethylamine, 2-ethoxy-1-propanol, 1-methylpiperazine, 1,3-propanediamine, 2-heptanamine, n,n-diethylethylenediamine, 1-methoxy-2-acetoxypropane, 4-methylpyridine, n,n'-diethyl-1,2-ethanediamine, 2,6-dimethylmorpholine, methylhexanoate, 1-propoxy-2-propanol. Plus particulièrement, ledit agent d'extraction organique est choisi parmi le groupe consistant en éthylamine, isopropylamine, n-propylamine, diethylamine, propanone, tetrahydrofurane, ethylacetate, butanone, 3-pentylamine, 2-methoxyethanamine, 1,4-dioxane, 3-pentanone, 2-pentanone, n-pentylamine, 1,3-dioxane, 1,2-diaminoethane, 1,2-propanediamine, 2-methoxyethanol, n-butylacetate, 1-ethoxy-2-propanol.

Ledit flux **G1h** comprenant 1,1,1,2,2-pentafluoropropane (HFC-245cb) peut être recyclé à l'étape a) du présent procédé.

Comme expliqué ci-dessus, le courant **G1i** comprenant trans-1,3,3,3-tetrafluoro-1-propène (HFO-1234zeE) et ledit agent d'extraction organique est distillé pour séparer l'agent d'extraction organique du trans-1,3,3,3-tetrafluoro-1-propène (HFO-1234zeE), avantageusement ledit agent d'extraction organique ainsi séparé est recyclé à l'étape c1). Le trans-1,3,3,3-tetrafluoro-1-propène (HFO-1234zeE) peut être incinéré ou purifié pour être utilisé ultérieurement ou pour être vendu.

Le courant **G1e** peut être purifié, par exemple par distillation extractive, pour éliminer du trans-1,3,3,3-tetrafluoro-1-propène (HFO-1234ze-E) éventuellement présent. Dans ce cas, ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide et hétérocycle ; ou ledit agent d'extraction organique est difluorodiethylsilane, triethylfluorosilane ou l'acide perfluorobutanoïque; de préférence parmi le groupe consistant en amine, éther, cétone, ester, alcool, aldéhyde, hétérocycle. Le point d'ébullition dudit agent d'extraction organique peut être compris entre 10 et 150°C. Ledit agent d'extraction organique peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propène (HFO-1234ze-E), dans ledit agent d'extraction organique à dilution infinie, P2 représente la pression de vapeur saturante de dudit au moins un des composés consistant en trans-1,3,3,3-tetrafluoro-1-propène (HFO-1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0. Ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du trans-1,3,3,3-tetrafluoro-1-propène (HFO-1234ze-E) dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0. Avantageusement, ledit agent d'extraction organique peut être éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, 1,4-dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, diethylcarbonate, n-butylacetate, 1-ethoxy-2-propanol, hexanal ; avantageusement ledit agent d'extraction organique est choisi parmi le groupe consistant en éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, 1,4-dioxane, trimethoxymethane, n-pentylamine, 1,3-dioxane, sec-butylacetate, 1,2-diaminoethane, n-butylacetate, 1-ethoxy-2-propanol, hexanal ; de préférence ledit agent d'extraction organique est choisi parmi le groupe consistant en éthylamine, isopropylamine, diethylether, dimethoxymethane, n-propylamine, diethylamine, diisopropylether, 2-ethoxy-2-methyl-propane, diethoxymethane, isopropylacetate, 3-pentylamine, 2-methoxyethanamine, tert-butylacetate, 1,4-dioxane, trimethoxymethane, n-pentylamine, 1,3-dioxane, sec-butylacetate, 1,2-diaminoethane, n-butylacetate, 1-ethoxy-2-propanol, hexanal.

Alternativement, le procédé comprend une étape c), subséquente à l'étape b5) ou à l'étape b7), dans laquelle le courant **G1c"** obtenu à l'étape b5) ou le courant **G1c""** obtenu à l'étape b7) comprend 2,3,3,3-tétrafluoropropène (HFO-1234yf), 1,1,1,2,2-pentafluoropropane (HFC-245cb) et trans-1,3,3,3-tetrafluoro-1-propène (HFO-1234zeE) ; et ledit courant **G1c"** ou **G1c""** est distillé pour former un courant **G1e'** comprenant 2,3,3,3-tétrafluoropropène (HFO-1234yf) et 1,1,1,2,2-pentafluoropropane (HFC-245cb) et un courant **G1f'** comprenant trans-1,3,3,3-tetrafluoro-1-propène (HFO-1234zeE), avantageusement le courant **G1c"** ou **G1c""** est distillé par distillation extractive.

Selon un mode de réalisation préféré, le courant **G1c"** ou **G1c""** est distillé par distillation extractive suivant les étapes :
c1') mise en contact dudit courant **G1c"** ou **G1c""** avec un agent d'extraction organique pour former un courant **G1g'**, et
c2') distillation extractive du courant **G1g'** pour former le flux **G1e'** comprenant 2,3,3,3-tetrafluoropropène (HFO-1234yf) et 1,1,1,2,2-pentafluoropropane (HFC-245cb), avantageusement en tête de colonne de distillation, et le courant **G1h'** comprenant trans-1,3,3,3-tetrafluoro-1-propène (HFO-1234zeE) et ledit agent d'extraction organique, avantageusement en bas de colonne de distillation.

Selon un mode de réalisation préféré, ledit agent d'extraction organique peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propène (HFO-1234ze-E) dans ledit agent d'extraction organique à dilution infinie, P2 représente la pression de vapeur saturante du trans-1,3,3,3-tetrafluoro-1-propène (HFO-1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8. Dans ce mode de réalisation, ledit agent d'extraction organique peut également avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 1,1,1,2,2-pentafluoropropane (HFC-245cb) dans ledit agent d'extraction organique à dilution infinie, P1 représente la pression de vapeur saturante du 1,1,1,2,2-pentafluoropropane (HFC-245cb), γ_{2,S} représente le coefficient d'activité du trans-1,3,3,3-tetrafluoro-1-propène (HFO-1234ze-E) dans ledit agent d'extraction organique à dilution infinie, P2 représente la pression de vapeur saturante du trans-1,3,3,3-tetrafluoro-1-propène (HFO-1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0. Dans ce mode de réalisation préféré, ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité dudit au moins un des composés consistant en trans-1,3,3,3-tetrafluoro-1-propène (HFO-1234ze-E) dans ledit agent d'extraction organique à dilution infinie, de préférence γ_{2,S} représente le coefficient d'activité dudit au moins un des composés consistant en trans-1,3,3,3-tetrafluoro-1-propène (HFO-1234ze-E), dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0. Ainsi, dans ce mode de réalisation préféré, ledit agent d'extraction organique peut être éthylamine, isopropylamine, diethylether, ethylmethylamine, 2-amino-2-methylpropane, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, 1-butylamine, ethylacetate, butanone, n-propylformate, 2,2-dimethoxypropane, 1-ethoxy-2-methylpropane, 1,2-dimethoxyethane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, ethylpropionate, 1,2-dimethoxypropane, 1,4-dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal ; avantageusement, éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, 1,4-dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal ; de préférence, éthylamine, isopropylamine, diethylether, n-propylamine, diethylamine, diethoxymethane, isopropylacetate, 3-pentylamine, 2-methoxyethanamine, tert-butylacetate, 1,4-dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal.

De préférence, le courant **G1e'** comprenant 2,3,3,3-tétrafluoropropène (HFO-1234yf) et 1,1,1,2,2-pentafluoropropane (HFC-245cb) peut être soumis à des étapes de purification ultérieures. Ainsi, le 2,3,3,3-tétrafluoropropène (HFO-1234yf) peut être séparé, de préférence par distillation du 1,1,1,2,2-pentafluoropropane (HFC-245cb) pour former un courant comprenant 2,3,3,3-tétrafluoropropène (HFO-1234yf) et un courant **G1i'** comprenant 1,1,1,2,2-pentafluoropropane (HFC-245cb), ledit courant **G1i'** étant recyclé à l'étape a). Le 2,3,3,3-tétrafluoropropène (HFO-1234yf) peut encore être soumis à des étapes de purification ultérieures afin d'obtenir un degré de pureté suffisant pour sa commercialisation. Par exemple, celui-ci peut être purifié, par exemple par distillation extractive, pour éliminer du trans-1,3,3,3-tetrafluoro-1-propène (HFO-1234ze-E) éventuellement présent.

De préférence, le courant **G1h'** comprenant trans-1,3,3,3-tetrafluoro-1-propène (HFO-1234zeE) et ledit agent d'extraction organique est séparé par distillation pour former un courant **G1j'** comprenant ledit agent d'extraction organique et un courant **G1k'** comprenant trans-1,3,3,3-tetrafluoro-1-propène (HFO-1234zeE). Le courant **G1j'** comprenant ledit agent d'extraction organique peut être recyclé à l'étape c1'). Le courant **G1k'** comprenant trans-1,3,3,3-tetrafluoro-1-propène (HFO-1234zeE) peut être soit purifié ou détruit par incinération.

Optionnellement ou non, si le courant **G1c** comprend des impuretés ayant un point d'ébullition inférieur à celui du 2,3,3,3-tetrafluoro-1-propène, celles-ci peuvent être éliminées par distillation. Lesdites impuretés ayant un point d'ébullition inférieur à celui du 2,3,3,3-tetrafluoro-1-propène peuvent être trifluorométhane (HFC-23), monofluorométhane (HFC-41), difluorométhane (HFC-32), pentafluoroéthane (HFC-125), 1,1,1-trifluoroéthane (HFC-143a), trifluoropropyne ou 1-chloro-pentafluoroéthane (CFC-115) ; et celles-ci peuvent être récupérées en tête de colonne de distillation. Le courant récupéré en bas de colonne de distillation peut ensuite être utilisé comme décrit ci-dessus pour le courant **G1c** dans l'étape b) et les étapes subséquentes.

Selon un autre mode de réalisation préféré, ledit courant liquide **L1** comprend tout ou partie des produits intermédiaires **B** et tout ou partie des produits secondaires **C**; et tout ou partie de ce courant **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C.** Ainsi, ledit courant liquide **L1** peut comprendre une partie des produits intermédiaires **B** et une partie des produits secondaires **C**; et tout ou partie de ce courant **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C.** Avantageusement, ladite basse température est comprise entre - 50°C et 15°C, de préférence entre -40°C et 10°C, en particulier entre-30°C et 0°C. Cette étape peut être effectuée de façon continue ou discontinue.

Ladite première phase **L1a** peut être recyclée à l'étape a).

Optionnellement ou non, ledit courant **G1d** formé à l'étape b2) peut être mélangé au courant liquide **L1** avant que tout ou partie de ce dernier soit porté à basse température.

Tout ou partie du 1,1,1,2,2-pentafluoropropane (HFC-245cb) peut aussi être contenu dans ledit courant liquide **L1** puis dans ladite seconde phase **L1b.**

Tout ou partie du 1,3,3,3-tetrafluoro-1-propène (HFO-1234ze) peut aussi être contenu dans ledit courant liquide **L1** puis dans ladite seconde phase **L1b.**

Tout ou partie du 2-chloro-3,3,3-trifluoropropène (HCFO-1233xf) peut aussi être contenu dans ledit courant liquide **L1** puis dans ladite seconde phase **L1b.**

Tout ou partie du E-1-chloro-3,3,3-trifluoro-1-propène (HCFO-1233zdE) peut aussi être contenu dans ledit courant liquide **L1** puis dans ladite seconde phase **L1b.**

Tout ou partie du 1,1,1,3,3-pentafluoropropane (HFC-245fa) peut aussi être contenu dans ledit courant liquide **L1** puis dans ladite seconde phase **L1b.**

De préférence, ladite seconde phase **L1b** peut comprendre 1,1,1,3,3-pentafluoropropane (HFC-245fa), E-1-chloro-3,3,3-trifluoro-1-propène (HCFO-1233zdE), 2-chloro-3,3,3-trifluoropropène (HCFO-1233xf), 1,3,3,3-tetrafluoro-1-propène (HFO-1234ze) et 1,1,1,2,2-pentafluoropropane (HFC-245cb).

Selon un mode de réalisation préféré, ladite seconde phase **L1b** est distillée pour récupérer un courant **L1c** comprenant 1,1,1,2,2-pentafluoropropane (HFC-245cb) et 1,3,3,3-tetrafluoro-1-propène (HFO-1234ze), avantageusement en tête de colonne de distillation, et un courant **L1d** comprenant 2-chloro-3,3,3-trifluoro-1-propène (HCFO-1233xf), E-1-chloro-3,3,3-trifluoro-1-propène (HCFO-1233zdE) et 1,1,1,3,3-pentafluoropropane (HFC-245fa) ; avantageusement en bas de colonne de distillation. Ledit courant **L1c** peut être recyclé à l'étape a). Optionnellement, ledit courant **L1c** peut être purifié pour séparer 1,1,1,2,2-pentafluoropropane (HFC-245cb) et 1,3,3,3-tetrafluoro-1-propène (HFO-1234ze). Ceci peut être effectué par distillation extractive tel qu'explicité ci-dessus en relation avec la séparation du courant **G1f.**

Selon un mode de réalisation préféré, ledit courant **L1d** peut être séparé pour former un flux comprenant 2-chloro-3,3,3-trifluoro-1-propène (HCFO-1233xf) et un courant comprenant E-1-chloro-3,3,3-trifluoro-1-propène (HCFO-1233zdE) et 1,1,1,3,3-pentafluoropropane (HFC-245fa). La séparation dudit courant **L1d** peut être effectuée par distillation extractive.

De préférence, ledit courant **L1d** peut être une composition azéotropique ou quasi-azéotropique comprenant 2-chloro-3,3,3-trifluoro-1-propène (HCFO-1233xf), E-1-chloro-3,3,3-trifluoro-1-propène (HCFO-1233zdE) et 1,1,1,3,3-pentafluoropropane (HFC-245fa).

De préférence, ladite séparation peut être effectuée par distillation extractive. Ladite distillation extractive dudit courant **L1d** comprend les étapes de :
- mise en contact dudit courant **L1d** avec un agent d'extraction organique pour former une composition **L1e,** et
- distillation extractive de la composition **L1e** pour former un flux **L1f** comprenant 2-chloro-3,3,3-trifluoro-1-propène (HCFO-1233xf), avantageusement en tête de colonne de distillation, et un courant **L1g** comprenant E-1-chloro-3,3,3-trifluoro-1-propène (HCFO-1233zdE) et 1,1,1,3,3-pentafluoropropane (HFC-245fa) et ledit agent d'extraction organique, avantageusement en bas de colonne de distillation.

De préférence, le courant **L1g** est ensuite séparé par distillation pour former un courant **L1h** comprenant ledit agent d'extraction organique et un courant **L1i** comprenant E-1-chloro-3,3,3-trifluoro-1-propène (HCFO-1233zdE) et 1,1,1,3,3-pentafluoropropane (HFC-245fa). Le courant **L1h** peut être recyclé pour être mis en contact avec un courant **L1d** pour former une composition **L1e.** Le courant **L1i** comprenant E-1-chloro-3,3,3-trifluoro-1-propène (HCFO-1233zdE) et 1,1,1,3,3-pentafluoropropane (HFC-245fa) peut être soit purifié soit détruit par incinération.

Selon un mode de réalisation préféré, ledit agent d'extraction organique mis en contact avec le courant **L1d** est un solvant choisi parmi le groupe consistant en hydrocarbure, hydrohalocarbure, alcool, cétone, amine, ester, éther, aldéhyde, nitrile, carbonate, sulfoxide, sulfate, thioalkyle, amide, hétérocycle et phosphate ou l'agent d'extraction organique est l'acide perfluorobutanoïque. Selon un mode de réalisation préféré, ledit agent d'extraction organique a un point d'ébullition compris entre 50 et 200°C. Selon un mode de réalisation préféré, ledit agent d'extraction organique a un facteur de séparation S₁,₂ supérieur ou égal à 1,1, ledit facteur de séparation étant calculé par la formule S₁,₂ = (γ_{1,s}*P1)/(γ_{2,s}*P2) dans laquelle
γ_{1,s} représente le coefficient d'activité du 2-chloro-3,3,3-trifluoropropène dans ledit agent d'extraction organique à dilution infinie,
P1 représente la pression de vapeur saturante du 2-chloro-3,3,3-trifluoropropène,
γ_{2,s} représente le coefficient d'activité du 1,1,1,3,3-pentafluoropropane (HFC-245fa) dans ledit agent d'extraction organique à dilution infinie,
P2 représente la pression de vapeur saturante du 1,1,1,3,3-pentafluoropropane (HFC-245fa);
avantageusement, le facteur de séparation S₁,₂ est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ;
et
ledit agent d'extraction organique a une capacité d'absorption C_{2,s} supérieure ou égale à 0,20, ladite capacité d'absorption étant calculé par la formule C_{2,s}= 1/(γ_{2,s}) dans laquelle γ_{2,s} représente le coefficient d'activité du 1,1,1,3,3-pentafluoropropane (HFC-245fa) dans ledit agent d'extraction organique à dilution infinie ; avantageusement, la capacité d'absorption C_{2,s} est supérieure ou égale à 0,40, de préférence supérieure ou égale à 0,60, plus préférentiellement supérieure ou égale à 0,80, en particulier supérieure ou égale à 1,0.

Ainsi, selon un mode de réalisation particulier, ledit agent d'extraction organique peut être choisi parmi le groupe consistant en éthanedial, propanone, methylacetate, methylglyoxal, ethylacetate, butanone, propionitrile, 1,4-dioxane, trimethoxymethane, 1,3-dioxane, 1,3,5-trioxane, 1,2-diaminoethane, 1-methoxy2-propanol, diethylcarbonate, 2-methoxyl-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate ; de préférence ledit agent d'extraction organique peut être choisi parmi le groupe consistant en propanone, methylacetate, ethylacetate, butanone, 1,4-dioxane, trimethoxymethane, 1,3-dioxane, 1,3,5-trioxane, 1,2-diaminoethane, 1-methoxy-2-propanol. De préférence, ce mode de réalisation particulier peut permettre de séparer efficacement 2-chloro-3,3,3-trifluoropropène et 1,1,1,3,3-pentafluoropropane (HFC-245fa).

Selon un mode de réalisation particulier, ledit agent d'extraction organique mis en contact avec le courant **L1d** peut avoir un facteur de séparation S₁,₂ supérieur ou égal à 1,1, ledit facteur de séparation étant calculé par la formule S_{1,2} = (γ_{1,s}*P1)/(γ_{2,s}*P2) dans laquelle
γ_{1,s} représente le coefficient d'activité du 2-chloro-3,3,3-trifluoropropène dans ledit agent d'extraction organique à dilution infinie,
P1 représente la pression de vapeur saturante du 2-chloro-3,3,3-trifluoropropène,
γ_{2,s} représente le coefficient d'activité du E-1-chloro-3,3,3-trifluoro-1-propène (HCFO-1233zdE) dans ledit agent d'extraction organique à dilution infinie,
P2 représente la pression de vapeur saturante du E-1-chloro-3,3,3-trifluoro-1-propène (HCFO-1233zdE);
avantageusement, le facteur de séparation S₁,₂ est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ;
et
ledit agent d'extraction organique peut avoir une capacité d'absorption C_{2,s} supérieure ou égale à 0,20, ladite capacité d'absorption étant calculé par la formule C_{2,s}= 1/(γ_{2,s}) dans laquelle γ_{2,s} représente le coefficient d'activité du E-1-chloro-3,3,3-trifluoro-1-propène (HCFO-1233zdE) dans ledit agent d'extraction organique à dilution infinie ; avantageusement, la capacité d'absorption C_{2,s} est supérieure ou égale à 0,40, de préférence supérieure ou égale à 0,60, plus préférentiellement supérieure ou égale à 0,8, en particulier supérieur ou égale à 1,0.

Ainsi, dans un mode de réalisation particulier, ledit agent d'extraction organique peut être choisi parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, -dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, 1,4-dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxyl-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; de préférence ledit agent d'extraction organique est choisi parmi le groupe consistant en diethylamine, propanone, methylacetate, tetrahydrofurane, ethylacetate, butanone, diethoxymethane, isopropylacetate, tert-butylacetate, 1,4-dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol. De préférence, ce mode de réalisation particulier peut permettre de séparer efficacement 2-chloro-3,3,3-trifluoropropène et E-1-chloro-3,3,3-trifluoro-1-propène (HCFO-1233zdE).

Selon un mode de réalisation préféré, pour favoriser l'élimination simultanée de E-1-chloro-3,3,3-trifluoro-1-propène (HCFO-1233zdE) et 1,1,1,3,3-pentafluoropropane (HFC-245fa) ; ledit agent d'extraction organique mis en contact avec le courant **L1d** peut être sélectionné parmi le groupe consistant en propanone, methylacetate, ethylacetate, butanone, 1,4-dioxane, trimethoxymethane, 1,3-dioxane, 1,2-diaminoethane, 1-methoxy2-propanol, diethylcarbonate, 2-methoxyl-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate. En particulier, ledit agent d'extraction organique peut être sélectionné parmi le groupe consistant en propanone, methylacetate, ethylacetate, butanone, 1,4-dioxane, trimethoxymethane, 1,3-dioxane, 1,2-diaminoethane, 1-methoxy2-propanol, 3-methoxy-1-butanol, diacetone alcohol.

Selon un mode de réalisation préféré, ledit courant **L1g** comprenant E-1-chloro-3,3,3-trifluoro-1-propène (HCFO-1233zdE), 1,1,1,3,3-pentafluoropropane (HFC-245fa) et ledit agent d'extraction organique peut être distillé pour séparer d'une part ledit agent d'extraction organique et d'autre part le E-1-chloro-3,3,3-trifluoro-1-propène (HCFO-1233zdE) et le 1,1,1,3,3-pentafluoropropane (HFC-245fa). De préférence, ledit agent d'extraction organique peut être recyclé.

Selon un mode de réalisation préféré, le flux **L1f** comprenant 2-chloro-3,3,3-trifluoro-1-propène (HCFO-1233xf) est recyclé à l'étape a).

Si des impuretés lourdes sont présentes dans ledit courant **L1d,** celui-ci peut être distillé préalablement à sa séparation pour éliminer celles-ci. Le courant **L1d** tel que décrit ci-dessus peut être récupéré en tête de colonne de distillation, les impuretés lourdes étant récupérées en bas de colonne de distillation. Les impuretés lourdes peuvent contenir par exemple 1,2-dichloro-3,3,3-trifluoropropène (HCFO-1223xd), cis-1-chloro-3,3,3-trifluoropropène, 2-chloro-1,1,1,3,3-pentafluoropropane, 2-chloro-1,3,3,3-tetrafluoropropène, des composés de formule (I).

Plus particulièrement, la composition de départ peut comprendre 1,1,2,3-tetrachloropropène, le 2,3,3,3,-tetrachloropropène, le 1,1,1,2,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, et le 1,1,1,3,3-pentafluoropropane, de préférence 1,1,1,2,3-pentachloropropane, 1,1,2,3,tetrachloropropène, du 1,1,1,2,2-pentafluoropropane et/ou du 2-chloro-3,3,3-trifluoro-1-propène, 1,2-dichloro-3,3,3-trifluoropropane ; en particulier 1,1,1,2,3-pentachloropropane (HCC-240db).

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 1.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bara, de préférence de 2 à 18 bara et plus particulièrement de 3 à 15 bara;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

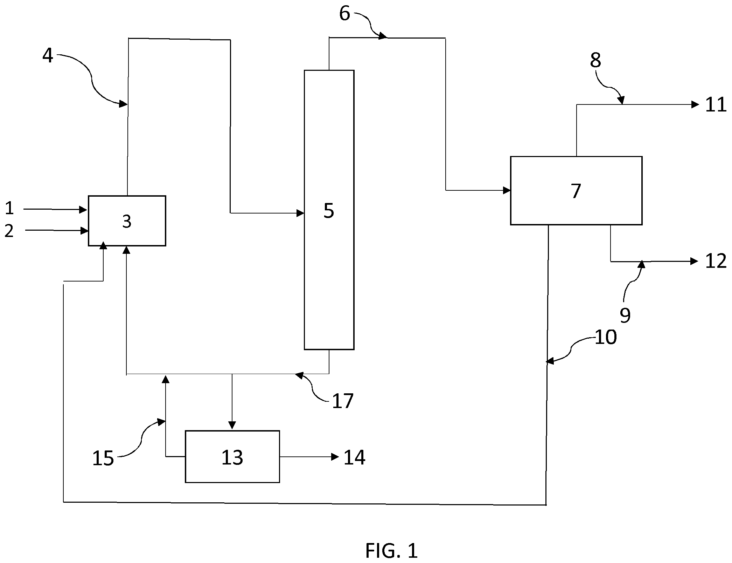

La Fig. 1 illustre schématiquement un dispositif mettant en œuvre un procédé de production du 2,3,3,3-tetrafluoropropène selon un mode de réalisation particulier de la présente invention. L'acide fluorhydrique 1 est mis en contact avec du 1,1,1,2,3-pentachloropropane (HCC-240db) 2 dans un ou plusieurs réacteurs 3. Le mélange A obtenu comprend HCl, du HF n'ayant pas réagi, 2,3,3,3-tétrafluoropropène (HFO-1234yf), 2-chloro-3,3,3-trifluoropropène (HCFO-1233xf), 1,1,1,2,2-pentafluoropropane (HFC-245cb), trans-1,3,3,3-tetrafluoro-1-propène (HFO-1234zeE), trans-1-chloro-3,3,3-trifluoro-1-propène (HCFO-1233zdE), cis-1-chloro-3,3,3-trifluoro-1-propène (HCFO-1233zdZ), cis-1,3,3,3-tetrafluoro-1-propène (HFO-1234zeZ), 1,1,1,3,3-pentafluoropropane (HFC-245fa), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), 1,1,1,3,3,3-hexafluoropropane (HFC-236fa) et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w est 0, w étant le nombre d'insaturations dans le composé de formule (II) considéré. Le mélange A est récupéré en sortie de réacteur et acheminé vers une colonne de distillation 5 par le conduit 4. Le courant liquide **L1** obtenu en pied de colonne de distillation 5 est acheminé soit partiellement soit totalement vers le dispositif de purification 13 via le conduit 17. Le courant gazeux **G1** obtenu en tête de colonne de distillation 5 est acheminé vers le dispositif de purification 7 via la conduite 6. Du dispositif de purification 7, un courant comprenant 2,3,3,3-tetrafluoro-1-propène est récupéré en 11 via le conduit 8. Un flux comprenant 1,1,1,2,2-pentafluoropropane (HFC-245cb), et optionnellement trans-1,3,3,3-tetrafluoro-1-propène (HFO-1234zeE), est obtenu également et recyclé vers le réacteur 3 par le conduit 10. Ledit flux comprenant 1,1,1,2,2-pentafluoropropane (HFC-245cb), et optionnellement trans-1,3,3,3-tetrafluoro-1-propène (HFO-1234zeE), contenu dans le conduit 10 peut éventuellement être mélangé avec le courant **L1** provenant de la conduite 15 ou 17 avant d'être injecté dans le réacteur 3. Enfin, un courant comprenant trans-1,3,3,3-tetrafluoro-1-propène (HFO-1234zeE) peut être récupéré en 12 par l'intermédiaire du conduit 9.

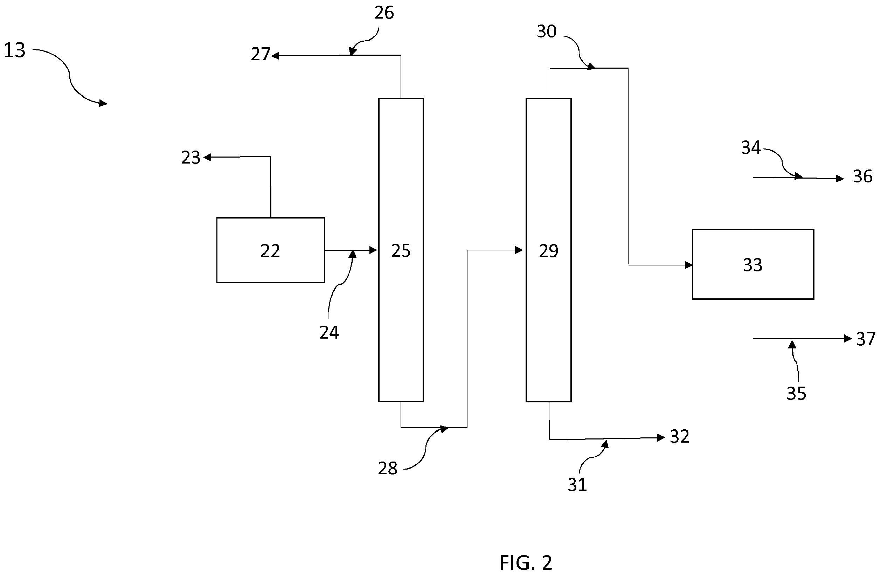

La Fig. 2 illustre schématiquement selon un mode de réalisation particulier de la présente invention un dispositif de purification 13. Un courant liquide **L1** obtenu en pied de colonne de distillation 5 (Figure 1) est acheminé vers le décanteur 22 ayant une température de -25°C. Le courant **L1a** est extrait et récupéré en 23 pour être recyclé vers le réacteur 3. Le courant **L1b** est acheminé vers la colonne de distillation 25 via le conduit 24. Le courant **L1c** est évacué en tête de colonne de distillation et récupérés en 27 via le conduit 26. Celui-ci peut être récupéré pour être recyclé vers le réacteur 3. Le courant **L1d** peut être acheminé vers la colonne de distillation 29 via le conduit 28 pour extraire en bas de colonne de distillation des impuretés lourdes éventuellement présentes et les acheminer vers un incinérateur 32 via le conduit 31. Le 2-chloro-3,3,3-trifluoropropène (HCFO-1233xf), E-1-chloro-3,3,3-trifluoro-1-propène (HCFO-1233zdE) et 1,1,1,3,3-pentafluoropropane (HFC-245fa) récupérés en tête de la colonne de distillation 29 sont acheminés vers un dispositif de purification 33 via le conduit 30. De ce dispositif de purification 33, le 2-chloro-3,3,3-trifluoropropène (HCFO-1233xf) peut être extrait en 36 via le conduit 34 et être recyclé vers le réacteur 3. Le dispositif de purification 33 peut être une distillation extractive. Le E-1-chloro-3,3,3-trifluoro-1-propène (HCFO-1233zdE) et 1,1,1,3,3-pentafluoropropane (HFC-245fa) peuvent être récupérés en 37 via le conduit 35 pour être incinérés ou purifiés.

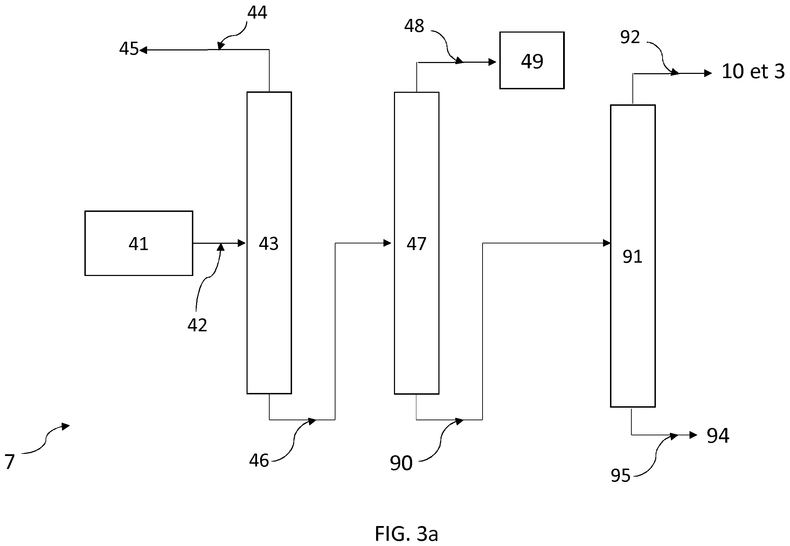

La Fig. 3a illustre schématiquement selon un mode de réalisation particulier de la présente invention un dispositif de purification 7. Le courant gazeux **G1** issu de la colonne de distillation 5 est acheminé en 41 puis vers la colonne de distillation 43 via le conduit 42. Le courant **G1a** est récupéré en tête de colonne de distillation 43 et traité en 45 via le conduit 44. Le dispositif 45 peut être un dispositif de purification du courant **G1a.** Le courant **G1b** récupéré en pied de colonne de distillation est acheminé vers la colonne de distillation 47 via le conduit 46. Un courant **G1c** est récupéré en tête de colonne de distillation pour être acheminé vers le dispositif de purification 49 via la conduite 48. Un courant **G1d** est récupéré en pied de colonne de distillation 47 pour être acheminé vers la colonne de distillation 91 via la conduite 90. La colonne de distillation 91 permet de former un courant **G1d'** récupéré en tête de colonne de distillation et un courant **G1d"** en pied de colonne de distillation. Le courant **G1d"** peut être envoyé vers un incinérateur 94 via la conduite 95. Le courant **G1d'** peut être acheminé vers la conduite 10 et le réacteur 3 (figure 1) via la conduite 92.

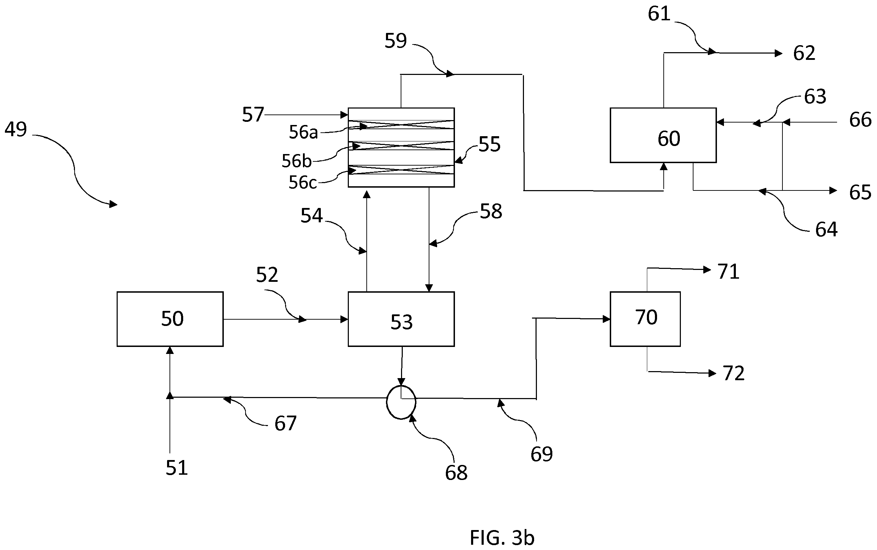

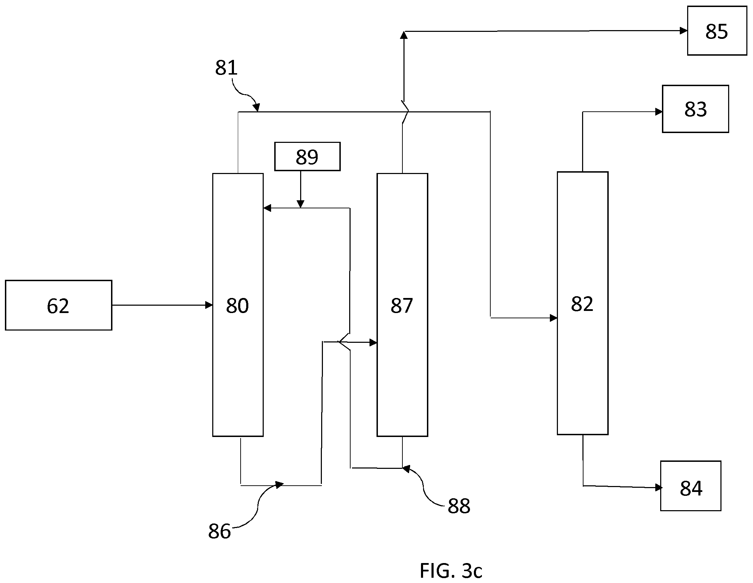

Le dispositif 49 est détaillé à la figure 3b. Celui-ci comprend un dispositif 50 permet la mise en contact entre le courant **G1c** et une solution d'acide fluorhydrique 51 ayant une concentration variant entre 65 et 75% en poids. Le dispositif 50 peut être par exemple un hydrolaveur. La mise en contact génère la formation d'un courant diphasique **G1c'** qui est acheminé vers un dispositif de stockage 53 par la conduite 52. La phase gazeuse du courant diphasique **G1c'** est acheminée par la conduite 54 vers la colonne d'absorption 55 comprenant 3 étages d'absorption 56a, 56b et 56c. La colonne d'absorption 55 est également alimentée par un flux aqueux 57. Dans ce mode de réalisation, le flux aqueux 57 alimente la colonne d'absorption 55 en tête de colonne d'absorption 55, c'est-à-dire au-dessus des trois étages d'absorption 56a-56c. Alternativement, le flux aqueux 57 peut alimenter la colonne d'absorption 55 au-dessus de chacun des étages d'absorption 56a-56c. Un courant gazeux **G1c"** est extrait en tête de colonne d'absorption 55 par la conduite 59 pour alimenter un dispositif de neutralisation 60. En outre, en pied de colonne d'absorption 55, une solution aqueuse d'acide fluorhydrique correspondant audit courant **G1c'"** est recyclée vers le dispositif de stockage 53 par la conduite 58. Le courant **G1c"** est neutralisé dans le dispositif de neutralisation 60 par une solution alcaline de NaOH à 20%. La solution alcaline 66 alimente le dispositif de neutralisation 60 par l'intermédiaire de la conduite 63. Le courant neutralisé est évacué par la conduite 61 pour être séché en 62. Le courant neutralisé et séché correspond au courant **G1c''''** selon le présent procédé. Celui-ci peut optionnellement être comprimé et liquéfié à une pression d'au plus 8 bara. Une solution alcaline usée 65 peut être extraite du dispositif de neutralisation 60 pour être soit recyclée via les conduites 64 et 63 ou évacuée via la conduite 64 pour traitement ultérieur. La phase liquide résultant du mélange de la phase liquide du courant diphasique et du courant **G1c'"** stocké dans le dispositif de stockage 53 est acheminée vers une colonne de distillation 70 via la pompe 68 et la conduite 69 pour former le courant **G2c** récupéré en tête de colonne de distillation 71 et le courant **G3c** récupéré en pied de colonne de distillation 72. La pompe 68 peut également être configurée pour acheminer la phase liquide résultant du mélange de la phase liquide du courant diphasique et du courant **G1c'"** stocké dans le dispositif de stockage 53 vers le dispositif 50 via la conduite 67. Le courant **G1c''''** obtenu en 62 est envoyé vers une colonne de distillation extractive 80 (Figure 3c). L'agent d'extraction organique 89 est mélangé au courant **G1c''''** avant d'entrer dans la colonne de distillation extractive 80. Le courant comprenant 2,3,3,3-tetrafluoropropène et 1,1,1,2,2-pentafluoropropane est récupéré en tête de colonne de distillation 80 pour être acheminé vers la colonne de distillation 82 via la conduite 81. Le courant comprenant 2,3,3,3-tetrafluoropropène et 1,1,1,2,2-pentafluoropropane est séparé par la colonne de distillation 82 pour former un courant 83 comprenant 2,3,3,3-tetrafluoropropène en tête de colonne de distillation et un courant 84 comprenant 1,1,1,2,2-pentafluoropropane en pied de colonne de distillation. Le courant comprenant trans-1,3,3,3-tetrafluoropropène et l'agent d'extraction organique est récupéré en pied de colonne de distillation 80 pour être acheminé vers la colonne de distillation 87 via la conduite 86. Un courant 85 comprenant trans-1,3,3,3-tetrafluoropropène est récupéré en tête de colonne de distillation. L'agent d'extraction organique est récupéré en pied de la colonne de distillation 87 et est recyclé vers la colonne de distillation 80 via la conduite 88.

## Revendications

1. Procédé de production et de purification du 2,3,3,3-tétrafluoropropène (HFO-1234yf) mis en œuvre à partir d'une composition de départ comprenant au moins un composé de formule (I) CH₍ₙ₊₂₎(X)ₘ-CHₚ(X)₍ₙ₊₁₎-CX₍₃₊ₚ₋ₘ₎ où X représente indépendamment F ou Cl; n, m, p sont indépendamment les uns des autres 0 ou 1 avec (n+m) = 0 ou 1, (n+p) = 0 ou 1 et (m-p) = 0 ou 1, au moins un X étant Cl; ledit procédé comprenant les étapes de :
a) mise en contact, en présence d'un catalyseur, de la composition de départ avec HF dans des conditions efficaces pour produire une composition **A** comprenant HCl, une partie du HF n'ayant pas réagi, 2,3,3,3-tétrafluoropropène (HFO-1234yf), des produits intermédiaires **B** consistant en 2-chloro-3,3,3-trifluoropropène (HCFO-1233xf), 1,1,1,2,2-pentafluoropropane (HFC-245cb), trans-1,3,3,3-tetrafluoro-1-propène (HFO-1234zeE) et des produits secondaires C consistant en trans-1-chloro-3,3,3-trifluoro-1-propène (HCFO-1233zdE), cis-1-chloro-3,3,3-trifluoro-1-propène (HCFO-1233zdZ), cis-1,3,3,3-tetrafluoro-1-propène (HFO-1234zeZ), 1,1,1,3,3-pentafluoropropane (HFC-245fa), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), 1,1,1,3,3,3-hexafluoropropane (HFC-236fa) et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w est 0, w étant le nombre d'insaturations dans le composé de formule (II) considéré ;
b) récupération de ladite composition **A** et purification, de préférence distillation, de celle-ci pour former et récupérer un courant gazeux **G1** comprenant HCl, 2,3,3,3-tétrafluoropropène (HFO-1234yf), une partie du HF n'ayant pas réagi, une partie des produits intermédiaires **B** et une partie des produits secondaires **C**; et un courant, de préférence liquide, **L1** comprenant une partie du HF n'ayant pas réagi, une partie des produits intermédiaires **B** et une partie des produits secondaires **C** ;
**caractérisé en ce que** ledit courant gazeux **G1** purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (HFO-1234yf), une partie du HF n'ayant pas réagi, ladite une partie des produits intermédiaires **B** et ladite une partie des produits secondaires C, avantageusement en bas de colonne de distillation ;
b2-1) distillation dudit courant **G1b** obtenu à l'étape b1) dans des conditions efficaces pour former un courant **G1c** gazeux comprenant 2,3,3,3-tétrafluoropropène (HFO-1234yf), une portion de ladite partie dudit HF n'ayant pas réagi, une portion de ladite partie des produits intermédiaires **B,** avantageusement en tête de colonne de distillation, et un courant **G1d** liquide comprenant une portion de ladite partie des produits intermédiaires **B** et ladite partie des produits secondaires **C** et une portion de ladite partie dudit HF n'ayant pas réagi, avantageusement en bas de colonne de distillation ;
b2-2) distillation dudit courant **G1d** obtenu à l'étape b2-1) dans des conditions efficaces pour former un courant **G1d'** comprenant ladite portion de ladite partie des produits intermédiaires **B,** ladite portion de ladite partie dudit HF n'ayant pas réagi et une portion de ladite partie des produits secondaires C comprenant cis-1,3,3,3-tetrafluoro-1-propène (HFO-1234zeZ), 1,1,1,3,3,3-hexafluoropropane (HFC-236fa) et une partie de trans-1-chloro-3,3,3-trifluoro-1-propène (HCFO-1233zdE) et 1,1,1,3,3-pentafluoropropane (HFC-245fa); et un courant **G1d"** comprenant une portion de ladite partie des produits secondaires **C** comprenant cis-1-chloro-3,3,3-trifluoro-1-propène (HCFO-1233zdZ), 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), et optionnellement un ou plusieurs composés de formule (II) CₙHₓF_{y}Cl_{z} dans laquelle n = 4, 5 ou 6, x est un nombre entier de 0 à 6, y est un nombre entier de 4 à 12, z est un nombre entier de 0 à 6, avec 2n = x+y+z si w est 1 ou 2n-2 = x+y+z si w est 2 ou 2n+2 = x+y+z si w est 0, w étant le nombre d'insaturations dans le composé de formule (II) considéré.

2. Procédé selon la revendication précédente **caractérisé en ce que** au moins 90% du 1,1,1,2,2-pentafluoropropane et du trans-1,3,3,3-tetrafluoropropène contenus dans le courant **G1d** sont récupérés dans le courant **G1d'**, avantageusement au moins 91%, de préférence au moins 92%, plus préférentiellement au moins 93%, en particulier au moins 94%, plus particulièrement au moins 95%, de manière privilégiée au moins 96%, de manière préférentiellement privilégiée au moins 97%, de manière particulièrement privilégiée au moins 98% du 1,1,1,2,2-pentafluoropropane et du trans-1,3,3,3-tetrafluoropropène contenus dans le courant **G1d** sont récupérés dans le courant **G1d'.**

3. Procédé selon l'une quelconque des revendications précédentes **caractérisé en ce que** le courant **G1d'** comprend de 60 à 70% en poids de 1,1,1,2,2-pentafluoropropane, de 1% à 5% en poids de trans-1,3,3,3-tetrafluoropropène et 10 à 15% en poids de 2-chloro-3,3,3-trifluoropropène sur base du poids total du courant **G1d'.**

4. Procédé selon l'une quelconque des revendications précédentes **caractérisé en ce que** au moins 85% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d'**, avantageusement au moins 90%, de préférence au moins 92%, plus préférentiellement au moins 93%, en particulier au moins 95%, plus particulièrement au moins 97% du cis-1,3,3,3-tetrafluoro-1-propène (HFO-1234zeZ) et du 1,1,1,3,3,3-hexafluoropropane (HFC-236fa) contenus dans le courant **G1d** sont récupérés dans le courant **G1d'.**

5. Procédé selon l'une quelconque des revendications précédentes **caractérisé en ce que** le courant **G1d'** comprend de 0,1 à 0,5% en poids de cis-1,3,3,3-tetrafluoro-1-propène (HFO-1234zeZ), de 1,0% à 5,0% en poids de 1,1,1,3,3,3-hexafluoropropane (HFC-236fa), 0,5 à 2,0% en poids de trans-1-chloro-3,3,3-trifluoro-1-propène (HCFO-1233zdE) et de 0,1 à 1,0% en poids de 1,1,1,3,3-pentafluoropropane (HFC-245fa) sur base du poids total du courant **G1d'.**

6. Procédé selon la revendication précédente **caractérisé en ce que** le courant **G1d'** comprend également 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe) en une teneur totale inférieure à 5% en poids sur base du poids total de **G1d'**, avantageusement inférieure à 4% en poids, de préférence inférieure à 3% en poids, plus préférentiellement inférieure à 2% en poids, en particulier inférieure à 1% en poids, plus particulièrement inférieure à 0,5% en poids sur base du poids total de **G1d'.**

7. Procédé selon l'une quelconque des revendications précédentes **caractérisé en ce que** le courant **G1d'** est recyclé à l'étape a).

8. Procédé selon l'une quelconque des revendications précédentes **caractérisé en ce qu'**il comprend également les étapes :
b3) mise en contact du courant **G1c** avec une solution aqueuse d'acide fluorhydrique de concentration supérieure à 40% pour former un courant **G1c'** diphasique comprenant 2,3,3,3-tétrafluoropropène (HFO-1234yf), de l'acide fluorhydrique, une portion de ladite partie des produits intermédiaires **B** et une portion de ladite partie des produits secondaires **C,**
b4) stockage dudit courant **G1c'** diphasique dans un réservoir tampon, ledit second courant diphasique étant constitué d'une phase liquide et d'une phase gazeuse,
b5) passage de la phase gazeuse dudit courant **G1c'** dans une colonne d'absorption alimenté à contre-courant par un flux aqueux pour former un courant **G1c"** comprenant 2,3,3,3-tétrafluoropropène (HFO-1234yf), une portion de ladite partie des produits intermédiaires **B** et une portion de ladite partie des produits secondaires C et un courant **G1c'"** comprenant HF ;
et optionnellement les étapes :
b6) neutralisation dudit courant **G1c"** obtenu à l'étape b5) par une solution aqueuse alcaline pour former un courant neutralisé, et
b7) séchage dudit courant neutralisé obtenu à l'étape b6), de préférence sur tamis moléculaire, pour former un courant neutralisé et séché **G1c''''.**

9. Procédé selon la revendication précédente **caractérisé en ce que** la solution aqueuse d'acide fluorhydrique utilisée à l'étape b3) est à une température comprise entre 0 à 30°C avant sa mise en contact avec le courant **G1c.**

10. Procédé selon l'une quelconque des revendications précédentes 8 et 9 **caractérisé en ce que**
le procédé comprend une étape c), subséquente à l'étape b5) ou à l'étape b7), dans laquelle le courant **G1c"** obtenu à l'étape b5) ou le courant **G1c''''** obtenu à l'étape b7) comprend 2,3,3,3-tétrafluoropropène (HFO-1234yf), 1,1,1,2,2-pentafluoropropane (HFC-245cb) et trans-1,3,3,3-tetrafluoro-1-propène (HFO-1234zeE) ; et ledit courant **G1c" ou G1c''''** est distillé pour former un courant **G1e'** comprenant 2,3,3,3-tétrafluoropropène (HFO-1234yf) et 1,1,1,2,2-pentafluoropropane (HFC-245cb) et un courant **G1h'** comprenant trans-1,3,3,3-tetrafluoro-1-propène (HFO-1234zeE) ;
avantageusement le courant **G1c"** ou **G1c''''** est distillé par distillation extractive ;
de préférence le courant **G1c"** ou **G1c''''** est distillé par distillation extractive par les étapes :
c1') mise en contact dudit courant **G1c" ou G1c''''** avec un agent d'extraction organique pour former un courant **G1g'**, et
c2') distillation extractive du courant **G1g'** pour former le flux **G1e'** comprenant 2,3,3,3-tetrafluoropropène (HFO-1234yf) et 1,1,1,2,2-pentafluoropropane (HFC-245cb), avantageusement en tête de colonne de distillation, et le courant **G1h'** comprenant trans-1,3,3,3-tetrafluoro-1-propène (HFO-1234zeE) et ledit agent d'extraction organique, avantageusement en bas de colonne de distillation.

11. Procédé selon l'une quelconque des revendications précédentes **caractérisé en ce que** ledit courant liquide **L1** comprend une partie des produits intermédiaires **B** et tout ou partie des produits secondaires **C,** et une partie du courant liquide **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C**; optionnellement ou non, ledit courant **G1d** formé à l'étape b2) est mélangé au courant liquide **L1** avant que ce dernier soit porté à basse température, avantageusement ladite première phase L1a est recyclée à l'étape a) ; avantageusement ladite seconde phase **L1b** est distillée pour récupérer un courant **L1c** comprenant 1,1,1,2,2-pentafluoropropane (HFC-245cb) et trans-1,3,3,3-tetrafluoro-1-propène (HFO-1234zeE), avantageusement en tête de colonne de distillation, et un courant **L1d** comprenant 2-chloro-3,3,3-trifluoro-1-propène (HCFO-1233xf), E-1-chloro-3,3,3-trifluoro-1-propène (HCFO-1233zdE) et 1,1,1,3,3-pentafluoropropane (HFC-245fa); avantageusement en bas de colonne de distillation, avantageusement ledit courant **L1c** est recyclé à l'étape a) ;
de préférence ledit courant **L1d** est séparé par distillation extractive pour former un flux comprenant 2-chloro-3,3,3-trifluoro-1-propène (HCFO-1233xf) et un courant comprenant E-1-chloro-3,3,3-trifluoro-1-propène (HCFO-1233zdE) et 1,1,1,3,3-pentafluoropropane (HFC-245fa).

## Patentansprüche

1. Verfahren zur Herstellung und Reinigung von 2, 3, 3, 3-Tetrafluorpropen (HFO-1234yf), das ausgehend von einer Ausgangszusammensetzung durchgeführt wird, die mindestens eine Verbindung der Formel (I) CH₍ₙ₊₂₎(X)ₘ-CHₚ (X) ₍ₙ₊₁₎-CX₍₃₊ₚ₋ₘ₎ umfasst, wobei X unabhängig F oder Cl darstellt; n, m und p unabhängig voneinander 0 oder 1 sind, wobei (n+m) = 0 oder 1, (n+p) = 0 oder 1 und (m-p) = 0 oder 1, wobei mindestens ein X Cl ist; wobei das Verfahren die Schritte umfasst:
a) In-Kontakt-Bringen, in Gegenwart eines Katalysators, der Ausgangszusammensetzung mit HF unter Bedingungen, die wirksam sind zur Herstellung einer Zusammensetzung **A,** umfassend HCl, einen nicht umgesetzten Teil der HF, 2,3,3,3-Tetrafluorpropen (HFO-1234yf), Zwischenprodukte **B,** bestehend aus 2-Chlor-3,3,3-trifluorpropen (HCFO-1233xf), 1,1,1,2,2-Pentafluorpropan (HFC-245cb), trans-1,3,3,3-Tetrafluor-1-propen (HFO-1234zeE), und Nebenprodukte **C,** bestehend aus trans-1-Chlor-3,3,3-trifluor-l-propen (HCFO-1233zdE), cis-1-Chlor-3,3,3-trifluor-l-propen (HCFO-1233zdZ), cis-1,3,3,3-Tetrafluor-l-propen (HFO-1234zeZ), 1,1,1,3,3-Pentafluorpropan (HFC-245fa), 2-Chlor-1,1,1,3,3-pentafluorpropan (HCFC-235da), 1,2-Dichlor-3,3,3-trifluorpropen (HCFO-1223xd), 2-Chlor-1,3,3,3-tetrafluorpropen (HCFO-1224xe), 1,1,1,3,3,3-Hexafluorpropan (HFC-236fa) und gegebenenfalls einer oder mehreren Verbindungen der Formel (II) CₙHₓF_{y}Cl_{z}, worin n = 4, 5 oder 6, x eine ganze Zahl von 0 bis 6 ist, y eine ganze Zahl von 4 bis 12 ist, z eine ganze Zahl von 0 bis 6 ist, wobei 2n = x+y+z, wenn w 1 ist, oder 2n-2 = x+y+z, wenn w 2 ist, oder 2n+2 = x+y+z, wenn w 0 ist, wobei w die Anzahl der Ungesättigtheiten in der betreffenden Verbindung der Formel (II) ist;
b) Gewinnung der Zusammensetzung **A** und deren Reinigung, vorzugsweise Destillation, zur Bildung und Gewinnung eines Gasstroms **Gl,** umfassend HCl, 2,3,3,3-Tetrafluorpropen (HFO-1234yf), einen nicht umgesetzten Teil der HF, einen Teil der Zwischenprodukte **B** und einen Teil der Nebenprodukte **C;** und eines, vorzugsweise flüssigen, Stroms **L1,** umfassend einen nicht umgesetzten Teil der HF, einen Teil der Zwischenprodukte **B** und einen Teil der Nebenprodukte **C;**
**dadurch gekennzeichnet, dass** der Gasstrom **Gl** durch die folgenden Schritte gereinigt wird:
bl) Destillation des Gasstroms **Gl** zur Gewinnung eines Stroms **Gla,** umfassend HCl, vorteilhafterweise am Kopf der Destillationskolonne, und eines Stroms **G1b,** umfassend 2,3,3,3-Tetrafluorpropen (HFO-1234yf), einen nicht umgesetzten Teil der HF, den einen Teil der Zwischenprodukte **B** und den einen Teil der Nebenprodukte **C,** vorteilhafterweise im Sumpf der Destillationskolonne;
b2-1) Destillation des im Schritt b1) erhaltenen Stroms **G1b** unter Bedingungen, die wirksam sind zur Bildung eines Gasstroms **G1c,** umfassend 2,3,3,3-Tetrafluorpropen (HFO-1234yf), einen Anteil des nicht umgesetzten Teils der HF, einen Anteil des Teils der Zwischenprodukte **B,** vorteilhafterweise am Kopf der Destillationskolonne, und eines Flüssigkeitsstroms **G1d,** umfassend einen Anteil des Teils der Zwischenprodukte **B** und den Teil der Nebenprodukte **C** sowie einen Anteil des nicht umgesetzten Teils der HF, vorteilhafterweise im Sumpf der Destillationskolonne;
b2-2) Destillation des im Schritt b2-1) erhaltenen Stroms **G1d** unter Bedingungen, die wirksam sind zur Bildung eines Stroms **G1d',** umfassend den Anteil des Teils der Zwischenprodukte **B,** den Anteil des nicht umgesetzten Teils der HF und einen Anteil des Teils der Nebenprodukte **C,** umfassend cis-1,3,3,3-Tetrafluor-1-propen (HFO-1234zeZ), 1,1,1,3,3,3-Hexafluorpropan (HFC-236fa) und einen Teil von trans-l-Chlor-3,3,3-trifluor-1-propen (HCFO-1233zdE) und 1,1,1,3,3-Pentafluorpropan (HFC-245fa); und eines Stroms **G1d'',** umfassend einen Anteil des Teils der Nebenprodukte **C,** umfassend cis-1-Chlor-3,3,3-trifluor-l-propen (HCFO-1233zdZ), 2-Chlor-1,1,1,3,3-Pentafluorpropan (HCFC-235da), 1,2-Dichlor-3,3,3-trifluorpropen (HCFO-1223xd), 2-Chlor-1,3,3,3-tetrafluorpropen (HCFO-1224xe) und gegebenenfalls eine oder mehrere Verbindungen der Formel (II) CₙHₓF_{y}Cl_{z}, worin n = 4, 5 oder 6, x eine ganze Zahl von 0 bis 6 ist, y eine ganze Zahl von 4 bis 12 ist, z eine ganze Zahl von 0 bis 6 ist, wobei 2n = x+y+z, wenn w 1 ist, oder 2n-2 = x+y+z, wenn w 2 ist, oder 2n+2 = x+y+z, wenn w 0 ist, wobei w die Anzahl der Ungesättigtheiten in der betreffenden Verbindung der Formel (II) ist.

2. Verfahren nach dem vorhergehenden Anspruch, **dadurch gekennzeichnet, dass** mindestens 90% des 1,1,1,2,2-Pentafluorpropans und des trans-1,3,3,3-Tetrafluorpropens, die im Strom **G1d** enthalten sind, im Strom **G1d'** gewonnen werden, vorteilhafterweise mindestens 91%, vorzugsweise mindestens 92%, stärker bevorzugt mindestens 93%, insbesondere mindestens 94%, spezieller mindestens 95%, bevorzugt mindestens 96%, besonders bevorzugt mindestens 97%, ganz besonders bevorzugt mindestens 98% des 1,1,1,2,2-Pentafluorpropans und des trans-1,3,3,3-Tetrafluorpropens, die im Strom **G1d** enthalten sind, im Strom **G1d'** gewonnen werden.

3. Verfahren nach einem der vorhergehenden Ansprüche, **dadurch gekennzeichnet, dass** der Strom **G1d'** 60 bis 70 Gew.-% 1,1,1,2,2-Pentafluorpropan, 1 bis 5 Gew.-% trans-1,3,3,3-Tetrafluorpropen und 10 bis 15 Gew.-% 2-Chlor-3,3,3-trifluorpropen, bezogen auf das Gesamtgewicht des Stroms **Gld',** umfasst.

4. Verfahren nach einem der vorhergehenden Ansprüche, **dadurch gekennzeichnet, dass** mindestens 85% des cis-1,3,3,3-Tetrafluor-1-propens (HFO-1234zeZ) und des 1,1,1,3,3,3-Hexafluorpropans (HFC-236fa), die im Strom **G1d** enthalten sind, im Strom **G1d'** gewonnen werden, vorteilhafterweise mindestens 90%, vorzugsweise mindestens 92%, stärker bevorzugt mindestens 93%, insbesondere mindestens 95%, spezieller mindestens 97% des cis-1,3,3,3-Tetrafluor-1-propens (HFO-1234zeZ) und des 1,1,1,3,3,3-Hexafluorpropans (HFC-236fa), die im Strom **G1d** enthalten sind, im Strom **G1d'** gewonnen werden.

5. Verfahren nach einem der vorhergehenden Ansprüche, **dadurch gekennzeichnet, dass** der Strom **G1d'** 0,1 bis 0,5 Gew.-% cis-1,3,3,3-Tetrafluor-1-propen (HFO-1234zeZ), 1,0 bis 5,0 Gew.-% 1,1,1,3,3,3-Hexafluorpropan (HFC-236fa), 0,5 bis 2,0 Gew.-% trans-1-Chlor-3,3,3-trifluor-1-propen (HCFO-1233zdE) und 0,1 bis 1,0 Gew.-% 1,1,1,3,3-Pentafluorpropan (HFC-245fa), bezogen auf das Gesamtgewicht des Stroms **Gld',** umfasst.

6. Verfahren nach dem vorhergehenden Anspruch, **dadurch gekennzeichnet, dass** der Strom **G1d'** außerdem 2-Chlor-1,1,1,3,3-pentafluorpropan (HCFC-235da), 1,2-Dichlor-3,3,3-trifluorpropen (HCFO-1223xd), 2-Chlor-1,3,3,3-tetrafluorpropen (HCFO-1224xe) in einem Gesamtgehalt von weniger als 5 Gew.-%, bezogen auf das Gesamtgewicht von **Gld',** vorteilhafterweise weniger als 4 Gew.-%, vorzugsweise weniger als 3 Gew.-%, stärker bevorzugt weniger als 2 Gew.-%, insbesondere weniger als 1 Gew.-%, spezieller weniger als 0,5 Gew.-%, bezogen auf das Gesamtgewicht von **G1d',** umfasst.

7. Verfahren nach einem der vorhergehenden Ansprüche, **dadurch gekennzeichnet, dass** der Strom **G1d'** zum Schritt a) rückgeführt wird.

8. Verfahren nach einem der vorhergehenden Ansprüche, **dadurch gekennzeichnet, dass** es außerdem die folgenden Schritte umfasst:
b3) In-Kontakt-Bringen des Stroms **G1c** mit einer wässrigen Flusssäurelösung mit einer Konzentration von mehr als 40% zur Bildung eines Zweiphasenstroms **G1c',** umfassend 2,3,3,3-Tetrafluorpropen (HFO-1234yf), Flusssäure, einen Anteil des Teils der Zwischenprodukte **B** und einen Anteil des Teils der Nebenprodukte **C,**
b4) Aufbewahren des Zweiphasenstroms **G1c'** in einem Pufferspeicher, wobei der zweite Zweiphasenstrom aus einer flüssigen Phase und einer Gasphase besteht,
b5) Leiten der Gasphase des Stroms **G1c'** in eine Absorptionskolonne, die im Gegenstrom mit einem wässrigen Fluss beschickt wird, zur Bildung eines Stroms **G1c'',** umfassend 2,3,3,3-Tetrafluorpropen (HFO-1234yf), einen Anteil des Teils der Zwischenprodukte **B** und einen Anteil des Teils der Nebenprodukte **C,** und eines Stroms **G1c''',** umfassend HF;
und gegebenenfalls die Schritte:
b6) Neutralisation des im Schritt b5) erhaltenen Stroms **G1c''** mit einer wässrigen alkalischen Lösung zur Bildung eines neutralisierten Stroms und
b7) Trocknen des in Schritt b6) erhaltenen neutralisierten Stroms, vorzugsweise auf einem Molekularsieb, zur Bildung eines neutralisierten und getrockneten Stroms **G1c''''.**

9. Verfahren nach dem vorhergehenden Anspruch, **dadurch gekennzeichnet, dass** die im Schritt b3) verwendete wässrige Flusssäurelösung eine Temperatur zwischen 0 und 30°C hat, bevor sie mit dem Strom **G1c** in Kontakt gebracht wird.

10. Verfahren nach einem der vorhergehenden Ansprüche 8 und 9, **dadurch gekennzeichnet, dass** das Verfahren einen Schritt c) nach Schritt b5) oder Schritt b7) umfasst, wobei der im Schritt b5) erhaltene Strom **G1c''** oder der im Schritt b7) erhaltene Strom **G1c''''** 2,3,3,3-Tetrafluorpropen (HFO-1234yf), 1,1,1,2,2-Pentafluorpropan (HFC-245cb) und trans-1,3,3,3-Tetrafluor-1-propen (HFO-1234zeE) umfasst; und der Strom **G1c''** oder **G1c''''** destilliert wird zur Bildung eines Stroms **G1e',** umfassend 2,3,3,3-Tetrafluorpropen (HFO-1234yf) und 1,1,1,2,2-Pentafluorpropan (HFC-245cb), und eines Stroms **G1h',** umfassend trans-1,3,3,3-Tetrafluor-1-propen (HFO-1234zeE);
wobei vorteilhafterweise der Strom **G1c''** oder **G1c''''** durch extraktive Destillation destilliert wird;
wobei vorzugsweise der Strom **G1c''** oder **G1c''''** durch extraktive Destillation durch die folgenden Schritte destilliert wird:
c1') In-Kontakt-Bringen des Stroms **G1c''** oder **G1c''''** mit einem organischen Extraktionsmittel zur Bildung eines Stroms **G1g'** und
c2') extraktive Destillation des Stroms **G1g'** zur Bildung des Flusses **Gle',** umfassend 2,3,3,3-Tetrafluorpropen (HFO-1234yf) und 1,1,1,2,2-Pentafluorpropan (HFC-245cb), vorteilhafterweise am Kopf der Destillationskolonne, und des Stroms **G1h',** umfassend trans-1,3,3,3-Tetrafluor-1-propen (HFO-1234zeE) und das organische Extraktionsmittel, vorteilhafterweise im Sumpf der Destillationskolonne.

11. Verfahren nach einem der vorhergehenden Ansprüche, **dadurch gekennzeichnet, dass** der flüssige Strom **L1** einen Teil der Zwischenprodukte **B** und alle oder einen Teil der Nebenprodukte **C** umfasst und ein Teil des flüssigen Stroms **L1** auf niedrige Temperatur, vorteilhafterwiese zwischen -50°C und 20°C, gebracht wird, um eine erste Phase **L1a,** umfassend einen nicht umgesetzten Teil der HF, und eine zweite Phase **L1b,** umfassend die Zwischenprodukte **B** und die Nebenprodukte **C,** zu bilden; wobei gegebenenfalls der im Schritt b2) gebildete Strom **G1d** mit dem Flüssigkeitsstrom **L1** gemischt wird, bevor der letztere auf niedrige Temperatur gebracht wird, wobei vorteilhafterweise die erste Phase L1a zum Schritt a) rückgeführt wird;
wobei vorteilhafterweise die zweite Phase **L1b** destilliert wird zur Gewinnung eines Stroms **L1c,** umfassend 1,1,1,2,2-Pentafluorpropan (HFC-245cb) und trans-1,3,3,3-Tetrafluor-1-propen (HFO-1234zeE), vorteilhafterweise am Kopf der Destillationskolonne, und eines Stroms **L1d,** umfassend 2-Chlor-3,3,3-trifluor-1-propen (HCFO-1233xf), E-1-Chlor-3,3,3-trifluor-1-propen (HCFO-1233zdE) und 1,1,1,3,3-Pentafluorpropan (HFC-245fa), vorteilhafterweise im Sumpf der Destillationskolonne, wobei vorteilhafterweise der Strom **L1c** zum Schritt a) rückgeführt wird;
wobei vorteilhafterweise der Strom **L1d** durch extraktive Destillation abgetrennt wird, um einen 2-Chlor-3,3,3-trifluor-1-propen (HCFO-1233xf) umfassenden Fluss und einen E-1-Chlor-3,3,3-trifluor-1-propen (HCFO-1233zdE) und 1,1,1,3,3-Pentafluorpropan (HFC-245fa) umfassenden Strom zu bilden.

## Claims

1. Process for the production and purification of 2,3,3,3-tetrafluoropropene (HFO-1234yf) carried out starting from a starting composition comprising at least one compound of formula (I) CH₍ₙ₊₂₎ (X)ₘ-CHₚ (X) ₍ₙ₊₁₎-CX₍₃₊ₚ₋ₘ₎, where X independently represents F or Cl; n, m and p are, independently of one another, 0 or 1 with n + m = 0 or 1, n + p = 0 or 1 and m - p = 0 or 1, at least one X being Cl; said process comprising the stages of:
a) bringing the starting composition into contact, in the presence of a catalyst, with HF under conditions effective in producing a composition **A** comprising HCl, a part of the unreacted HF, 2,3,3,3-tetrafluoropropene (HFO-1234yf), intermediate products **B** consisting of 2-chloro-3,3,3-trifluoropropene (HCFO-1233xf), 1,1,1,2,2-pentafluoropropane (HFC-245cb) and trans-1,3,3,3-tetrafluoro-1-propene (HFO-1234zeE) and byproducts **C** consisting of trans-1-chloro-3,3,3-trifluoro-1-propene (HCFO-1233zdE), cis-1-chloro-3,3,3-trifluoro-1-propene (HCFO-1233zdZ), cis-1,3,3,3-tetrafluoro-1-propene (HFO-1234zeZ), 1,1,1,3,3-pentafluoropropane (HFC-245fa), 2-chloro-1,1,1,3,3-pentafluoropropane (HCFC-235da), 1,2-dichloro-3,3,3-trifluoropropene (HCFO-1223xd), 2-chloro-1,3,3,3-tetrafluoropropene (HCFO-1224xe), 1,1,1,3,3,3-hexafluoropropane (HFC-236fa) and optionally one or more compounds of formula (II) CₙHₓF_{y}Cl_{z} in which n = 4, 5 or 6, x is an integer from 0 to 6, y is an integer from 4 to 12, z is an integer from 0 to 6, with 2n = x + y + z if w is 1 or 2n - 2 = x + y + z if w is 2 or 2n + 2 = x + y + z if w is 0, w being the number of unsaturations in the compound of formula (II) considered;
b) recovery of said composition **A** and purification, preferably distillation, of the latter in order to form and recover a gas stream **G1** comprising HCl, 2,3,3,3-tetrafluoropropene (HFO-1234yf), a part of the unreacted HF, a part of the intermediate products **B** and a part of the byproducts **C;** and a stream, which is preferably liquid, **L1** comprising a part of the unreacted HF, a part of the intermediate products **B** and a part of the byproducts **C;**
**characterized in that** said gas stream **G1** is purified by the following stages:
b1) distillation of the gas stream **G1** in order to recover a stream **G1a** comprising HCl, advantageously at the distillation column top, and a stream **G1b** comprising 2,3,3,3-tetrafluoropropene (HFO-1234yf), a part of the unreacted HF, said a part of the intermediate products **B** and said a part of the byproducts **C,** advantageously at the distillation column bottom;
b2-1) distillation of said stream **G1b** obtained in stage b1) under conditions effective in forming a gas stream **G1c** comprising 2,3,3,3-tetrafluoropropene (HFO-1234yf), a portion of said part of said unreacted HF, a portion of said part of the intermediate products **B,** advantageously at the distillation column top, and a liquid stream **G1d** comprising a portion of said part of the intermediate products **B** and said part of the byproducts **C** and a portion of said part of said unreacted HF, advantageously at the distillation column bottom;
b2-2) distillation of said stream **G1d** obtained in stage b2-1) under conditions effective in forming a stream **G1d'** comprising said portion of said part of the intermediate products **B,** said portion of said part of said unreacted HF and a portion of said part of the byproducts **C** comprising cis-1,3,3,3-tetrafluoro-1-propene (HFO-1234zeZ), 1,1,1,3,3,3-hexafluoropropane (HFC-236fa) and a part of trans-1-chloro-3,3,3-trifluoro-1-propene (HCFO-1233zdE) and 1,1,1,3,3-pentafluoropropane (HFC-245fa); and a stream **G1d''** comprising a portion of said part of the byproducts **C** comprising cis-1-chloro-3,3,3-trifluoro-1-propene (HCFO-1233zdZ), 2-chloro-1,1,1,3,3-pentafluoropropane (HCFC-235da), 1,2-dichloro-3,3,3-trifluoropropene (HCFO-1223xd), 2-chloro-1,3,3,3-tetrafluoropropene (HCFO-1224xe), and optionally one or more compounds of formula (II) CₙHₓF_{y}Cl_{z} in which n = 4, 5 or 6, x is an integer from 0 to 6, y is an integer from 4 to 12, z is an integer from 0 to 6, with 2n = x + y + z if w is 1 or 2n - 2 = x + y + z if w is 2 or 2n + 2 = x + y + z if w is 0, w being the number of unsaturations in the compound of formula (II) considered.

2. Process according to the preceding claim, **characterized in that** at least 90% of the 1,1,1,2,2-pentafluoropropane and of the trans-1,3,3,3-tetrafluoropropene contained in the stream **G1d** are recovered in the stream **G1d';** advantageously, at least 91%, preferably at least 92%, more preferably at least 93%, in particular at least 94%, more particularly at least 95%, favorably at least 96%, preferably favorably at least 97%, particularly favorably at least 98%, of the 1,1,1,2,2-pentafluoropropane and of the trans-1,3,3,3-tetrafluoropropene contained in the stream **G1d** are recovered in the stream **G1d'.**

3. Process according to either one of the preceding claims, **characterized in that** the stream **G1d'** comprises from 60% to 70% by weight of 1,1,1,2,2-pentafluoropropane, from 1% to 5% by weight of trans-1,3,3,3-tetrafluoropropene and from 10% to 15% by weight of 2-chloro-3,3,3-trifluoropropene, based on the total weight of the stream **G1d'.**

4. Process according to any one of the preceding claims, **characterized in that** at least 85% of the cis-1,3,3,3-tetrafluoro-1-propene (HFO-1234zeZ) and of the 1,1,1,3,3,3-hexafluoropropane (HFC-236fa) contained in the stream **G1d** are recovered in the stream **G1d';** advantageously, at least 90%, preferably at least 92%, more preferably at least 93%, in particular at least 95%, more particularly at least 97%, of the cis-1,3,3,3-tetrafluoro-1-propene (HFO-1234zeZ) and of the 1,1,1,3,3,3-hexafluoropropane (HFC-236fa) contained in the stream **G1d** are recovered in the stream **G1d'.**

5. Process according to any one of the preceding claims, **characterized in that** the stream **G1d'** comprises from 0.1% to 0.5% by weight of cis-1,3,3,3-tetrafluoro-1-propene (HFO-1234zeZ), from 1.0% to 5.0% by weight of 1,1,1,3,3,3-hexafluoropropane (HFC-236fa), from 0.5% to 2.0% by weight of trans-1-chloro-3,3,3-trifluoro-1-propene (HCFO-1233zdE) and from 0.1% to 1.0% by weight of 1,1,1,3,3-pentafluoropropane (HFC-245fa), based on the total weight of the stream **G1d'.**

6. Process according to the preceding claim, **characterized in that** the stream **G1d'** also comprises 2-chloro-1,1,1,3,3-pentafluoropropane (HCFC-235da), 1,2-dichloro-3,3,3-trifluoropropene (HCFO-1223xd) and 2-chloro-1,3,3,3-tetrafluoropropene (HCFO-1224xe) in a total content of less than 5% by weight, based on the total weight of **G1d'**, advantageously less than 4% by weight, preferably less than 3% by weight, more preferably less than 2% by weight, in particular less than 1% by weight, more particularly less than 0.5% by weight, based on the total weight of **G1d'.**

7. Process according to any one of the preceding claims, **characterized in that** the stream **G1d'** is recycled in stage a).

8. Process according to any one of the preceding claims, **characterized in that** it also comprises the stages:
b3) bringing the stream **G1c** into contact with an aqueous hydrofluoric acid solution with a concentration of greater than 40% in order to form a two-phase stream **G1c'** comprising 2,3,3,3-tetrafluoropropene (HFO-1234yf), hydrofluoric acid, a portion of said part of the intermediate products **B** and a portion of said part of the byproducts **C,**
b4) storage of said two-phase stream **G1c'** in a holding tank, said second two-phase stream consisting of a liquid phase and of a gas phase,
b5) passage of the gas phase of said stream **G1c'** into an absorption column fed countercurrentwise with an aqueous flow in order to form a stream **G1c''** comprising 2,3,3,3-tetrafluoropropene (HFO-1234yf), a portion of said part of the intermediate products **B** and a portion of said part of the byproducts **C** and a stream **G1c'''** comprising HF;
and optionally the stages:
b6) neutralization of said stream **G1c''** obtained in stage b5) with an aqueous alkaline solution in order to form a neutralized stream, and
b7) drying of said neutralized stream obtained in stage b6), preferably over a molecular sieve, in order to form a neutralized and dried stream **G1''''.**

9. Process according to the preceding claim, **characterized in that** the aqueous hydrofluoric acid solution used in stage b3) is at a temperature of between 0 and 30°C before it is brought into contact with the stream **G1c.**

10. Process according to either one of the preceding Claims 8 and 9, **characterized in that** the process comprises a stage c), subsequent to stage b5) or to stage b7), in which the stream **G1c''** obtained in stage b5) or the stream **G1''''.** obtained in stage b7) comprises 2,3,3,3-tetrafluoropropene (HFO-1234yf), 1,1,1,2,2-pentafluoropropane (HFC-245cb) and trans-1,3,3,3-tetrafluoro-1-propene (HFO-1234zeE); and said stream **G1c''** or **G1''''.** is distilled in order to form a stream **G1e'** comprising 2,3,3,3-tetrafluoropropene (HFO-1234yf) and 1,1,1,2,2-pentafluoropropane (HFC-245cb) and a stream **G1h'** comprising trans-1,3,3,3-tetrafluoro-1-propene (HFO-1234zeE);
advantageously, the stream **G1c''** or **G1''''.** is distilled by extractive distillation;
preferably, the stream **G1c''** or **G1c''''** is distilled by extractive distillation by the stages:
c1') bringing said stream **G1c''** or **G1c''''** into contact with an organic extraction agent in order to form a stream **G1g'**, and
c2') extractive distillation of the stream **G1g'** in order to form the flow **G1e'** comprising 2,3,3,3-tetrafluoropropene (HFO-1234yf) and 1,1,1,2,2-pentafluoropropane (HFC-245cb), advantageously at the distillation column top, and the stream **G1h'** comprising trans-1,3,3,3-tetrafluoro-1-propene (HFO-1234zeE) and said organic extraction agent, advantageously at the distillation column bottom.

11. Process according to any one of the preceding claims, **characterized in that** said liquid stream **L1** comprises a part of the intermediate products **B** and all or part of the byproducts **C,** and a part of the liquid stream **L1** is brought to low temperature, advantageously between -50°C and 20°C, in order to form a first phase **L1a** comprising a part of the unreacted HF and a second phase **L1b** comprising said intermediate products **B** and said byproducts **C;** optionally or not, said stream **G1d** formed in stage b2) is mixed with the liquid stream **L1** before the latter is brought to low temperature; advantageously, said first phase **L1a** is recycled in stage a) ;
advantageously, said second phase **L1b** is distilled in order to recover a stream **L1c** comprising 1,1,1,2,2-pentafluoropropane (HFC-245cb) and trans-1,3,3,3-tetrafluoro-1-propene (HFO-1234zeE), advantageously at the distillation column top, and a stream **L1d** comprising 2-chloro-3,3,3-trifluoro-1-propene (HCFO-1233xf), E-1-chloro-3,3,3-trifluoro-1-propene (HCFO-1233zdE) and 1,1,1,3,3-pentafluoropropane (HFC-245fa), advantageously at the distillation column bottom; advantageously, said stream **L1c** is recycled in stage a);
preferably, said stream **L1d** is separated by extractive distillation in order to form a flow comprising 2-chloro-3,3,3-trifluoro-1-propene (HCFO-1233xf) and a stream comprising E-1-chloro-3,3,3-trifluoro-1-propene (HCFO-1233zdE) and 1,1,1,3,3-pentafluoropropane (HFC-245fa).
